# EUROPEAN PATENT APPLICATION

(11) **EP 2 312 482 A1**
(43) Date of publication of application: **20.04.2011**
(21) Application number: 11150574.9
(22) Date of filing: 22.03.2005
(51) Int. Cl.: G06F 19/00

(54) **Systems and methods for providing a stem cell bank**

(30) Priority: 26.03.2004 US 556683 P
(62) Divisional of application: 05725995.4
(71) Applicant: Celgene Corporation, Summit New Jersey 07901 (US)
(72) Inventor: The designation of the inventor has not yet been filed
(74) Representative: Piening, Niklas

(57) **Abstract**

Methods, computer systems, and computer program products for maintaining a stem cell registry comprising information about a plurality of stem cell units. A donor is enrolled in the stem cell registry. A stem cell unit from the donor is characterized. Information about the stem cell unit, obtained by the characterizing, is recorded in the stem cell registry. Computer readable media comprising a plurality of data records. One or more respective data records in the plurality of data records comprises (i) a collection identifier number that uniquely corresponds to a stem cell donation, (ii) a cord blood cell count associated with the stem cell donation, and (iii) a placenta blood cell count associated with the stem cell donation. Additional computer readable media comprising a plurality of data records. One or more respective data records in the plurality of data records comprises (i) a cord blood cell count associated with a stem cell donation, (ii) a placenta blood cell count associated with the stem cell donation, and (iii) an indication of at least two stem cell transplant units in the stem cell donation.

## Description

### RELATED APPLICATION

The present application claims benefit under 35 U.S.C. § 119(e), of United States Provisional Patent Application No. 60/556,683 entitled "Systems and Methods for Providing a Stem Cell Bank," filed March 26, 2004, which is hereby incorporated by reference in its entirety.

### 1. INTRODUCTION

This invention relates to the implementation and maintenance of a stem cell bank, or a stem cell producing facility, whereby the advantages of having multiple units from a single donor can be realized. More particularly, this invention relates to methods, computer systems, and computer program products for facilitating a stem cell bank in which individual donations to the bank comprise multiple transplant units of stem cells from cord blood, placenta, and/or other sources. Similarly, the methods, systems and products of the invention can be used to more efficiently procure, process, bank and dispense stem cells for transplantation, and other diagnostic or therapeutic cellular therapies.

### 2. BACKGROUND OF THE INVENTION

### 2.1. STEM CELL APPLICATIONS

There is considerable interest in the identification, isolation and generation of human stem cells. Human stem cells are totipotent or pluripotent precursor cells capable of generating a variety of mature human cell lineages. This ability serves as the basis for the cellular differentiation and specialization necessary for organ and tissue development. Further, such cells are used for bone marrow transplantation (BMT). BMT is an often used means of therapy for a variety of malignant and genetic diseases, comprising chemotherapy-resistant malignancies and genetic blood diseases. For certain diseases (*e.g.,* leukemias and specific immunodeficiencies) it is the only proven treatment for long-term patient survival. For other diseases (*e.g.,* autoimmune diseases) it could offer the hope of a long-term cure.

If BMT is needed, the patient's family is tested for suitably HLA-matched members. However, there is only a 1 in 4 chance that a patient's sibling will be a suitable match. If a family member is not identified, then a search can be performed through the National Marrow Donor Program (NMDP) or similar registry for an HLA-matched, unrelated volunteer bone marrow donor. However, the chances of finding a suitably matched unrelated donor are approximately 30 percent for Caucasians, and significantly less for other ethnic groups. Also, the search process can take from 3-6 months and is often very expensive. Although it is possible to use bone marrow from a volunteer donor (which is frequently done), there are potentially a number of serious side-effects from this process. The most serious side-effect is a condition called graft-versus-host disease (GVHD), in which cells in the transplanted bone marrow graft start to attack the patient. GVHD is a major cause of death when it occurs, and it occurs 60-90 percent of the time in unrelated BMT. Due to the problems of a lack of donors and the high incidence of GVHD, researchers have looked to alternate sources of stem cells for transplantation.

Work that was begun in the early 1980s revealed that cord blood, the leftover blood remaining in the umbilical cord and placenta after the birth of a child, was comparable to bone marrow in terms of transplant potential. Cord blood offers a number of advantages over bone marrow. With over 4 million births per year in the United States, the potential donors were essentially unlimited. Over the past six years clinical use of cord blood has shown that more ethnic minority patients have been able to be transplanted, the incidence and severity of GVHD has been significantly reduced, and the costs of transplant have been considerably less than with BMT. In addition to BMT applications, scientific evidence suggests that stem cells can be used to repopulate many, if not all, tissues and restore physiologic and anatomic functionality. The application of stem cells in tissue engineering, gene therapy delivery, and cell therapeutics is also advancing rapidly.

### 2.2. METHODS FOR OBTAINING STEM CELLS

Mammalian stem cells have been obtained from a variety of sources For example, embryonic stem cells, embryonic germ cells, adult stem cells or other committed stem cells or progenitor cells are known. Certain types of stem cells have not only been isolated and characterized but have also been cultured under conditions that allow limited differentiation. A basic problem remains, however, in that obtaining sufficient quantities and populations of human stem cells capable of differentiating into the many different desired cell types is nearly impossible. The provision of matched stem cell units of sufficient quantity and quality remains a challenge despite the fact that these are important for the treatment of a wide variety of disorders, comprising malignancies, inborn errors of metabolism, hemoglobinopathies, and immunodeficiencies.

Umbilical cord blood ("cord blood") is a viable alternative source to other hematopoietic progenitor sources (*e.g.,* bone marrow and mobilized peripheral blood) for related and unrelated allogenic hematopoietic stem cell (HSC) / progenitor cell (HPC) transplantation. See, for example, Broxmeyer et al., 2003, PNAS 100, 645-650. For example, stem cells from cord blood are routinely cryopreserved for use in hematopoietic reconstitution, a widely used therapeutic procedure used in bone marrow and other related transplantations. See *e.g.,* Boyse et al., U.S. Patent No. 5,004,681, "Preservation of Fetal and Neonatal Hematopoietic Stem and Progenitor Cells of the Blood"; Boyse et al., U.S. Patent No. 5,192,553, "Isolation and preservation of fetal and neonatal hematopoietic stem and progenitor cells of the blood and methods of therapeutic use." In fact, high-efficiency recovery of functional hematopoietic progenitor and stem cells have been obtained from human cord blood cryopreserved for 15 years. See, for example, Broxmeyer et al., 2003, PNAS 100, 645-650.

Traditional techniques for the collection of cord blood are based on the use of a needle or cannula, which is used with the aid of gravity to drain cord blood from (*e.g.,* exsanguinate) the placenta (Boyse et al., U.S. Patent No. 5,192,553, issued March 9, 1993; Boyse et al., U.S. Patent No. 5,004, 681, issued April 2, 1991; Anderson, U.S. Patent No. 5,372,581, entitled "Method and apparatus for placental blood collection," issued December 13, 1994; Hessel et al., U.S. Patent No. 5,415,665, entitled "Umbilical cord clamping, cutting, and blood collecting device and method," issued May 16, 1995). The needle or cannula is usually placed in the umbilical vein and the placenta is gently massaged to aid in draining cord blood from the placenta.

A major limitation of stem cell procurement from cord blood has been the frequently inadequate volume of cord blood obtained, resulting in insufficient cell numbers to effectively reconstitute bone marrow after transplantation. While some work has gone into expanding such cell populations using culturing techniques, the drawback of currently available methods for such *ex vivo* expansion of stem cell populations is that the techniques are labor-intensive , time-consuming, often expensive, and may result in low yields of stem cells.

To address the shortcomings in the art, as to the expansion of stem cells recovered from cord blood, attention has turned to additional sources of stem cells. For example, Hariri (PCT applications PCT/US02/04282 entitled "Post-partum mammalian placenta, its use and placental stem cells therefrom," published August 22, 2002 as WO 02/064755 A2, and PCT/US01/46506 entitled "Method of collecting Placental Stem Cells," published June 13, 2002) describe methods of extracting and recovering embryonic like stem cells, comprising, but not limited to pluripotent or multipotent stem cells, from an exsanguinated human placenta post-partum. In Hariri, a placenta is treated to remove residual umbilical cord blood cells as well as other placenta cells by perfusing an exsanguinated placenta, preferably with an anticoagulant solution to flush out residual cells. The residual cells and perfusion liquid from the exsanguinated placenta are collected, and the embryonic-like stem cells are separated from the residual cells and perfusion liquid. United States Patent Application serial number 10/004,942, published September 5, 2002 as U.S. publication number 2002/0123141 A1 provides a method of collecting embryonic-like stem cells from a placenta that has been treated to remove residual cord blood cells as well as other placenta cells. In the method, the placenta, which has been drained of cord blood, is perfused with an anticoagulant perfusion solution and/or other type of perfusate such as saline solution to flush out residual cells and embryonic-like stem cells from the drained placenta. In some instances, the placenta is perfused with perfusate for a period of time such as between thirty minutes and five hours, between one hour and four hours, or more than four hours. In one embodiment, the placenta is perfused for about two hours. Then, residual cells and embryonic-like stem cells and perfusion solution from the drained placenta is collected. United States Patent Application serial number 10/076,180, published February 13, 2003 as U.S. publication number 2003/0032179 A1 describes conditions for incubating an isolated mammalian placenta for between 6 and 24 hours in order to obtain embryonic-like stem cells and other multipotent stem cells from the placenta. However, in some embodiments, the placenta is not incubated. These techniques are not only unique but they also exploit the over four million per year successful U.S. births by utilizing placenta and other tissues that would ordinarily be discarded.

### 2.3. STEM CELL BANKS

The advances in the procurement and use of stem cells has led to a need for storage repositories, also termed stem cell banks, for storing such cells. Known stem cell banks can be classified into two categories, private banks and public banks. Private banks (*e.g.,* family banks) store harvested stem cells for a donor's family and provide a unit of the donated stem cells back to the donor family if needed. Public banks have been established to provide typed, anonymous transplant units to the general public based on genetic matching with needy potential recipients. A general discussion of various ethical issues relating to cord-blood banks is provided in Jeremy Sugarman et al., "Ethical Aspects of Banking Placental Blood for Transplantation," 274 JAMA 22, pp. 1783-85, Dec., 13, 1995. In addition, Moore et al. (U.S. Patent No. 5,993,387 entitled "Computer-based mixed-use registry of placental and umbilical cord stem cells," issued November 30, 1999) disclosed a mixed use stem cell bank that allows for both typed, anonymous transplant units as well as exclusive family use and retention of cord stem cell units.

Although known stem cell banks serve an important function, they have drawbacks. Such banks are designed to provide an entire cord blood unit to a patient once specific conditions have been satisfied. A cord blood unit is the blood collected from a single placenta and umbilical cord. See, for example, the Cord Blood Stem Cell Act of 2003, introduced into both the United States House and the United States Senate in 2003. The conditions that must be satisfied to dispense a cord blood unit to a patient are blood bank specific. For example, if the stem cell bank is a private bank, the donor must authorize the release of the cord blood unit to a patient and, typically, this patient has a familial relationship with the donor. In the case of public stem cell banks, specific screening criteria, such as human leukocyte antigen matching between the donor and the recipient, is used to determine whether to dispense a cord blood unit to a patient. Thus, once a cord blood unit is donated to a recipient by the stem cell bank, the stem cell bank no longer has a sufficient amount of the blood having the exact characteristics of the donated unit to treat the same patient or another patient at a later date. Some stem cell banks have contemplated remedying this problem in the art by using clonal expansion techniques to substantially increase the number of nucleated cells in the donated sample. As used herein, a substantial increase in the number of a nucleated cells (*e.g.,* stem cells) in a population of cells (*e.g.,* a donated sample) can be defined as a twenty-five percent or greater increase in the number of nucleated cells in the population.

The practice of shipping the entire cord blood unit (or nearly the entire cord blood unit) to a recipient is unsatisfactory for many reasons. For instance, in a public stem cell bank, such a practice depletes the stem cell bank of stem cell units, thereby reducing the diversity of the blood units in the bank and consequently reducing the chances that a good match will be found for subsequent patients. In the case of a private family stem cell bank, the practice of donating an entire cord blood unit to a family member leaves other family members vulnerable due to the depletion of stem cells likely to have good matching characteristics (*e.g.,* HLA type). Further, there are many instances where the patient may need a second transplant and where it would be beneficial to retain sufficient additional stem cells for this second transplant that are autonomous with (*e.g.,* from the same donor) the cells used in the first transplant. However, while the practice of donating an entire cord blood unit has significant drawbacks, it has been largely unavoidable to date because patients have traditionally needed the entire cord blood unit in order to obtain enough stem cells to attempt to remedy their malignancy. Advancements in the field of stem cell collection, such as those disclosed by Hariri (PCT application PCT/US02/04282 published August 22, 2002 as WO 02/064755 A2) where stem cells obtained from the umbilical cord are supplemented with placenta perfusate, however, are increasing the number of stem cells that can be collected from a single donor to the point where it is no longer necessary to allocate to a patient an entire unit collected from such a donor. Despite the advancements in stem cell collection leading to a significant increase in the number of stem cells in a cord blood unit from a single donor, stem cell banks have not provided methods for dividing a cord blood unit into a plurality of units, where each respective unit in the plurality of units is sufficient to treat a patient without substantial clonal expansion of the respective unit and then providing one or more units from the plurality of units to a patient while retaining one or more units in the plurality of units for treatment of the same patient or a different patient without a requirement of substantial clonal expansion of such units. The present invention addresses, in part, these and other shortcomings present in the known art.

### 3. SUMMARY OF THE INVENTION

The invention includes systems and methods for enabling a stem cell bank to provide individual transplant units of stem cells to patients. Advantageously, such stem cell transplant units can be from a single donor. Further the present invention provides systems and method for maintaining client anonymity while maintaining access to essential medical information comprising patient profiles, immunologic tests, haplotyping, and the like.

### 3.1. PROVIDING MULTIPLE STEM CELL TRANSPLANT UNITS

One embodiment of the present invention provides a method of maintaining a stem cell registry comprising information about a plurality of stem cell units. In the method, a donor is enrolled in the stem cell registry and a stem cell unit from the donor is characterized. Then, information about the stem cell unit obtained by the characterizing step is recorded in the stem cell registry. In some embodiments, the stem cell unit comprises a plurality of stem cell transplant units from a single donor.

In some embodiments, the number of nucleated stem cells in a stem cell transplant unit (or a placental stem cell transplant unit) is a function of the weight of a patient and the number of cells per kilogram of patient weight that are to be delivered to such a patient. In other embodiments, the number of nucleated stem cells in a stem cell transplant unit (or a placental stem cell transplant unit) is not a function of the weight of the patient. Rather, in such embodiments, the weight of the patient or other characteristics of the patient are used to determine how many stem cell transplant units (or placental stem cell transplant units) are to be delivered to the patient. In some embodiments of the present invention, each stem cell transplant unit comprises between 150 x 10⁶ and 10000 x 10⁶ nucleated cells, between 300 x 10⁶ and 5000 x 10⁶ nucleated cells, or between 500 x 10⁶ and 3000 x 10⁶ nucleated cells. Each such stem cell transplant unit need not be equally divided units.

Once a stem cell registry has been constructed, patient type information (*e.g.,* HLA type) is received and used as a basis for searching through the plurality of stem cell units for a stem cell unit having matching type information. When a match is found between a stem cell unit in the registry and the patient, a number of stem cell transplant units from the matching stem cell unit is allocated to the patient. Furthermore, the number of stem cell transplant units of the matching stem cell unit available in the registry is decremented by the number of stem cell transplant units allocated to the patient. In typical embodiments, only a single stem cell transplant unit is allocated to the patient.

In some instances, the number of nucleated cells in a stem cell transplant unit is not a function of the body weight of the patient. In such embodiments, the number of stem cell transplant units allocated to a patient from a matching stem cell unit is can be a function of the number of nucleated stem cells in each donated stem cell transplant unit and the weight of the patient (or some other characteristic of the patient such as disease type, disease state, age, *etc.*)*.* Examples of stem cell transplant units in accordance with such embodiments include between 150 x 10⁶ and 10000 x 10⁶ nucleated cells, between 300 x 10⁶ and 5000 x 10⁶ nucleated cells, or between 500 x 10⁶ and 3000 x 10⁶ nucleated cells.

In some embodiments, the donor is mammalian and the stem cell unit from the donor comprises stem cells obtained from a post-partum placenta of the donor that has been exsanguinated and perfused. In some embodiments, the stem cell unit from the donor comprises stem cells obtained from the cord blood of the donor in addition to the exsanguinated and perfused post-partum placenta of the donor. Further, the stem cells from the cord blood of the donor form one or more stem cell transplant units and the stem cells obtained from the post-partum placenta of the donor form one or more different stem cell transplant units. In some embodiments, the stem cell registry comprises at least 100 stem cell units or, more preferably, at least 1000 stem cell units.

In some embodiments of the present invention, a donor pays a fee to store stem cells. Such a donor can be referred to as a private donor. Subsequently, a request is received from the private donor or a family member of the private donor for the stem cell unit. Next, one or more stem cell transplant units in the at least two stem cell transplant units are allocated to the private donor or the family member of the private donor. Remaining stem cell transplant units in the stem cell unit are then reserved for future use by the private donor or the family of the private donor. In some embodiments, a donor does not pay a fee to donate stem cells. In such instances the donor is a public donor and patients other than the donor may use the donor's cells. In a preferred embodiment, the stem cell transplant units that are allocated include one stem cell transplant unit from cord blood and one stem cell transplant unit from the placenta. In another preferred embodiment, the stem cell transplant units that are allocated include one stem cell transplant unit from cord blood and one stem cell transplant unit that comprises cord blood mixed with stem cells from placenta perfusate. In embodiments where a stem cell transplant unit is obtained entirely from placenta perfusate, the stem cell transplant unit can be termed a placental stem cell transplant unit in order to denote that the source of origin of the stem cells was the placenta.

Another aspect of the present invention provides a computer program product for use in conjunction with a computer system. The computer program product comprises a computer readable storage medium and a computer program mechanism embedded therein. The computer program mechanism comprises a stem cell database comprising information about a plurality of stem cell units and a stem cell tracking module. The stem cell tracking module includes a data entry routine that comprises instructions for enrolling a donor in the stem cell database, instructions for receiving a characterization of a stem cell unit from the donor, and instructions for recording information about the stem cell unit in the stem cell database. This information includes information about a plurality of placental stem cell transplant units or, more generally, a plurality of stem cell transplant units in the stem cell unit

Still another aspect of the present invention provides a computer system comprising a stem cell database that includes information about a plurality of stem cell units and a stem cell tracking module. The computer system comprises one or more computers. The stem cell tracking module includes a data entry routine that comprises (i) instructions for enrolling a donor in the stem cell database, (ii) instructions for receiving a characterization of a stem cell unit from the donor, (iii) and instructions for recording information about the stem cell unit in the stem cell database. This information includes information about a plurality of stem cell transplant units in the stem cell unit.

### 3.2. MAINTAINING DONOR ANONYMITY

In addition to providing registries in which individual stem cell donations comprise multiple doses, the present invention provides methods for maintaining client anonymity. This is accomplished by using a system that has two applications, a customer relationship management (CRM) application and a laboratory information management system (LIMS) application. The CRM application maintains information about a donor, comprising the name of the donor, donor contact information, the name of the donor's parents, and contact information of the donor's parents, and medical history records. The CRM application further stores a collection identifier number that is uniquely associated with the donor. The CRM application, however, does not store stem cell characterization information, such as infectious disease results and HLA blood type. Rather, the LIMS application, which is isolated from the CRM application either through electronic (password / permission) or physical (separate network / building) means, maintains the stem cell characterization information. The LIMS application does not track patient names and cannot access the patient's names. Rather, donations are tracked by the collection identifier number. In this way, lab workers entering sensitive assay results cannot learn the identity of the donors unless they have been granted special access privileges. Further, administrators of the CRM application cannot access the lab results on the LIMS application unless they have special privileges.

In some embodiments, the CRM application and the LIMS application are each hosted by different server means.

One embodiment of the present invention in accordance with this aspect of the invention comprises a computer program product for use in conjunction with a computer system. The computer program product comprises a computer readable storage medium and a computer program mechanism embedded therein. The computer program mechanism comprises a customer relationship management database comprising information about a plurality of donors as well as a customer relationship management application. The customer relationship management application includes (i) instructions for enrolling a donor in the customer relationship management database and (ii) instructions for assigning a collection identifier number to the donor. The computer program mechanism further includes a stem cell database comprising information about a plurality of stem cell units and a stem cell tracking module that includes a data entry routine for entering information into the stem cell database. This data entry routine includes (i) instructions for receiving the collection identifier number for the donor and (ii) instructions for receiving a characterization of a stem cell unit from the donor. In some embodiments, the characterization includes a characterization of stem cells from at least two different origins of the donor.

In some embodiments, a first origin in the at least two different origins is cord blood of the donor and a second origin in the at least two different origins is the placenta of the donor. In some instances, placenta has been exsanguinated and perfused in order to produce stem cells. In some embodiments, the customer relationship management database includes a respective data entry for each donor in the plurality of donors. Each such respective data entry comprises a name of the donor, donor contact information, the collection identifier number associated with the donor, a cord blood cell count, and a placenta blood cell count. In some embodiments, the stem cell database comprises a respective data entry for each donor in the plurality of donors, where each respective data entry comprises the collection identifier number associated with the donor, a characterization of stem cells from a first origin of the donor, and a characterization of stem cells from a second origin of the donor.

In some embodiments, the customer relationship management database or the stem cell database is a flat file, a relational database, an on-line analytical processing database, or a hierarchical on-line analytical processing data cube. In some instances, the customer relationship management database or the stem cell database does not have an explicitly defined hierarchy. In some embodiments, the customer relationship management database or the stem cell database includes a relational star schema.

In some embodiments of the present invention, the laboratory information management system further includes (i) instructions for receiving type information of a patient and (ii) instructions for searching through the plurality of stem cell units for a stem cell unit having type information that matches the type information (*e.g.,* HLA blood type) of the patient. In some embodiments, the laboratory information management system further includes instructions for receiving a search privilege and the instructions for searching are not performed when the search privilege is not received. In some embodiments, when a match is found between a stem cell unit in the plurality of stem cell units and the patient, the method further comprises granting the matching stem cell unit, or stem cell transplants units from the matching stem cell unit, to the patient when the matching stem cell unit is public.

Still another embodiment of the invention provides a computer system comprising a central processing unit, a network interface card for communicating with a remote computer, and a memory coupled to the central processing unit. This memory stores a customer relationship management database comprising information about a plurality of donors. The customer relationship management application includes (i) instructions for enrolling a donor in the customer relationship management database, (ii) instructions for uniquely associating a collection identifier number to the donor, and (iii) instructions for transmitting the collection identifier number to a stem cell tracking module that is hosted by the remote computer.

Yet another embodiment of the invention comprises a central processing unit, a network interface card for communicating with a remote computer, and a memory, coupled to the central processing unit. The memory stores a stem cell database comprising information about a plurality of stem cell units and a stem cell tracking module. The stem cell tracking module includes a data entry routine that comprises (i) instructions for receiving a collection identifier number from the remote computer such that the collection identifier number is uniquely associated with a donor, (ii) instructions for receiving a characterization of a stem cell unit from the donor such that the characterization includes a characterization of stem cells from at least two different origins of the donor, (iii) and (iii) instructions for updating the stem cell tracking module with the characterization of a stem cell unit from the donor.

Still another embodiment of the invention comprises a central processing unit, a network interface card for communicating with a remote computer, and a memory coupled to the central processing unit. The memory stores a stem cell database comprising information about a plurality of placental stem cell units and a stem cell tracking module. The stem cell tracking module includes a data entry routine that comprises (i) instructions for receiving a collection identifier number from the remote computer such that the collection identifier number is uniquely associated with a donor, (ii) instructions for receiving a characterization of a plurality of placental stem cell units from the donor, and (iii) instructions for updating the stem cell tracking module with the characterization of a placental stem cell unit from the donor.

### 3.3. INFORMATION HOUSED IN COMPUTER READABLE MEDIA

Some embodiments of the present invention provide novel information housed in computer readable media that is used to facilitate a stem cell bank. For instance, one embodiment of the present invention provides information housed in computer readable media that can be used to maintain client anonymity in a stem cell bank. One example of this embodiment of the present invention is a computer program product for use in conjunction with a computer system. The computer program product comprises a computer readable storage medium and a computer program mechanism embedded therein. The computer program mechanism comprises a plurality of data records. One or more respective data records in the plurality of data records comprises (i) a collection identifier number that uniquely corresponds to a stem cell donation, (ii) a cord blood cell count associated with the stem cell donation, and (iii) a placenta blood cell count associated with the stem cell donation. In some instances, stem cell donation originates from a mammal and the placental blood cell count represents a number of cells obtained from a post-partum placenta of the mammal after the placenta has been exsanguinated and perfused.

Another embodiment of the present invention provides information housed in computer readable media that is used to enable a cord blood bank in which individual donations comprise multiple stem cell transplant units. One example of this embodiment is a computer program product for use in conjunction with a computer system. The computer program product comprises a computer readable storage medium and a computer program mechanism embedded therein. The computer program mechanism comprises a plurality of data records. One or more respective data records in the plurality of data records comprises: (i) a cord blood cell count associated with a stem cell donation, (ii) a placenta blood cell count associated with the stem cell donation, and (iii) an indication of at least two stem cell transplant units in the stem cell donation. In some embodiments, the stem cell donation originates from a mammal and the placental blood cell count represents a number of cells obtained from a post-partum placenta of the mammal after the placenta has been exsanguinated and perfused.

### 3.4. DEFINITIONS

As used herein, the term "allogeneic cell" refers to a "foreign" cell, *i.e.,* a heterologous cell (*i.e.,* a "non-self' cell derived from a source other than the placental donor) or autologous cell (*i.e.,* a "self" cell derived from the placental donor) that is derived from an organ or tissue other than the placenta.

As used herein, the term "cord blood derived stem cell" includes cord blood-derived progenitor cells, unless otherwise specifically noted.

As used herein, the term "cord blood unit" refers to the blood collected from a single placenta and umbilical cord of a single donor.

As used herein, the term "exsanguinated" or "exsanguination" when used with respect to the placenta, refers to the removal and/or draining of substantially all blood from the placenta. In accordance with the present invention, exsanguination of the placenta can be achieved by, for example, and not by way of limitation, draining, gravity induced efflux, massaging, squeezing, pumping, *etc*. In a preferred embodiment, exsanguination of the placenta may further be achieved by perfusing, rinsing or flushing the placenta with a fluid that may or may not contain agents, such as anticoagulants, to aid in the exsanguination of the placenta.

As used herein, the term "perfuse" or "perfusion" refers to the act of pouring or passaging a fluid over or through an organ or tissue, preferably the passage of fluid through an organ or tissue with sufficient force or pressure to remove any residual cells, *e.g.,* nonattached cells form the organ or tissue. As used herein, the term "perfusate" refers to the fluid collected following its passage through an organ or tissue. Perfusing fluids used within the invention and the techniques of perfusing are described in U.S, patent application No. 10/076,180, filed February 13, 2002, which is hereby incorporated by reference.

As used herein, the term "placental stem cell transplant unit" is (a) any portion of a placental stem cell unit or (b) an entire placental stem cell unit, provided that the portion of the placental stem cell unit or the entire placental stem cell unit comprises a therapeutically effective amount of stem cells.

As used herein, a "placental stem cell unit" refers to a population comprising stem cells, from a single donor, that have been derived from an exsanguinated placenta. It is specifically contemplated that, in event of multiple births, multiple stem cell populations can be obtained from more than one placenta from a single donor. Populations comprising stem cells from a single donor that have been derived from an exsanguinated placenta can be obtained by methods disclosed within, for example, United States Patent Application No. 10/076,180, filed February 13, 2002, and United States Patent Application No. 10/004,942, filed December 5, 2001, both of which are hereby incorporated by reference in their entireties.

As used herein, the term "progenitor cell" refers to a cell that is committed to differentiate into a specific type of cell or to form a specific type of tissue.

As used herein, the term "stem cell" refers to a master cell that can differentiate to form the specialized cells of tissues and organs. A stem cell is a developmentally pluripotent or multipotent cell. A stem cell can divide to produce two daughter stem cells, or one daughter stem cell and one progenitor ("transit") cell, which then proliferates into the tissue's mature, fully formed cells.

As used herein, the term "stem cell transplant unit" is (a) any portion of a stem cell unit or (b) an entire stem cell unit, provided that the portion of the stem cell unit or the entire stem cell unit comprises a therapeutically effective amount of stem cells.

As used herein, a "stem cell unit" refers to a population comprising stem cells, from a single donor, that have been derived from a single patient source such as, for example, an exsanguinated placenta, cord blood, fetal tissue, bone marrow, or any other tissue or any other organ of the single donor from which stem cells can be derived.

As used herein, a "therapeutically effective amount of stem cells" is an amount of stem cells that is sufficient to cause a physiological effect such as, for example, (a) an amount sufficient, upon administration to a patient, to effect engraftment of any kind or (b) an amount sufficient, upon administration to a patient, to effect hematopoietic reconstitution.

### 4. BRIEF DESCRIPTION OF THE DRAWINGS

The above and other objects, features, and advantages of the present invention are further described in the detailed description that follows, with reference to the drawings by way of non-limiting exemplary embodiments of the present invention, wherein like reference numerals represent similar parts of the present invention throughout the several views of the drawings and wherein:
Fig. 1 depicts a computer system for hosting a laboratory information management system application in accordance with an embodiment of the present invention;
Fig. 2 depicts a computer system for hosting a customer relationship management application in accordance with an embodiment of the present invention;
Fig. 3 illustrates a flow diagram for obtaining a stem cell unit comprising multiple stem cell transplant units from a donor in accordance with an embodiment of the present invention;
Fig. 4 illustrates a flow diagram for dispensing a stem cell transplant unit in accordance with an embodiment of the present invention;
Fig. 5 illustrates a method for obtaining a stem cell donation while maintaining donor anonymity bank in accordance with an embodiment of the present invention; and
Fig. 6 illustrates a method for obtaining a stem cell match for a patient in a stem cell bank while maintaining donor anonymity in accordance with an embodiment of the present invention.
Fig. 7 illustrates an exemplary system in accordance with one embodiment of the present invention.

### 5. DETAILED DESCRIPTION OF THE INVENTION

One embodiment of the present invention is directed toward methods of collecting multiple stem cells transplant units from a given donor. An advantage of such collection schemes is that a portion of the stem cells from the given donor can be provided to a patient while retaining portions of the original stem cell donation from the given donor. Another is that more nucleated cells are available to the patient in need then previous conventional cord blood only banking. The retained portions referred to above can then be used for other beneficial purposes, such as for treating siblings of the patient, other family members of the patients, or, in fact, other patients that have no relationship to the original patient.

Another embodiment of the present invention provides donor anonymity. In such embodiments, a laboratory information management system (LIMS) application provides a stem cell database that records assay test results for a stem cell donation, such as human immunodeficiency virus (HIV) status, but does not record the patients name or other indicia. Rather, each donation is assigned a collection identifier number. Separately, a customer relationship management (CRM) application stores donor information, comprising names and addresses. The CRM application also stores collection identifier numbers. Thus, it is possible for a CRM application user to access confidential assay results stored by the LIMS application using the collection identifier numbers. However, in the present invention, access to the LIMS application by the CRM application is restricted. In this way patient anonymity is preserved.

### 5.1. GENERAL ARCHITECTURE

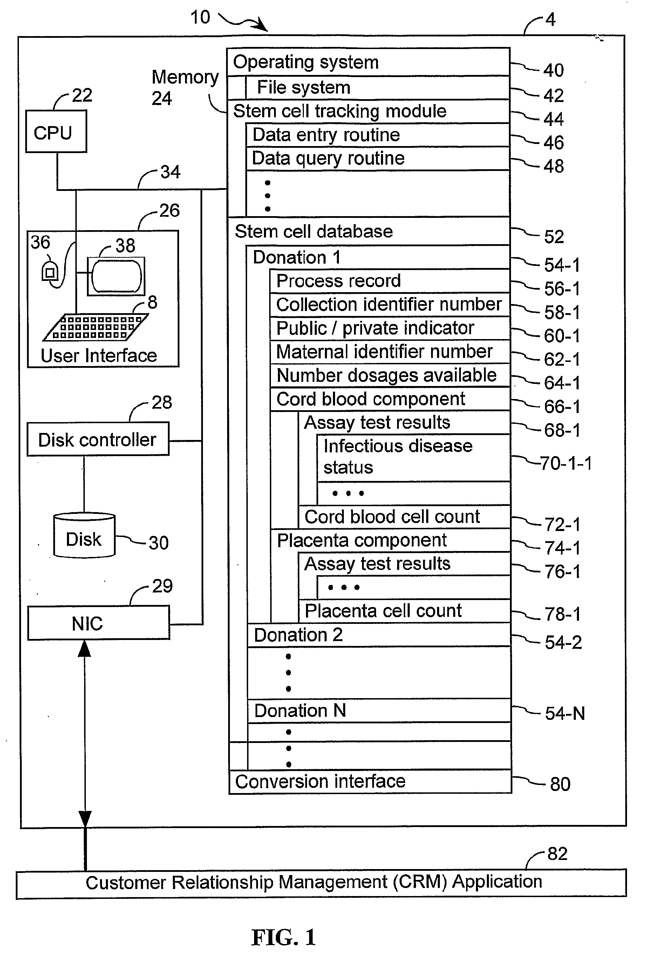

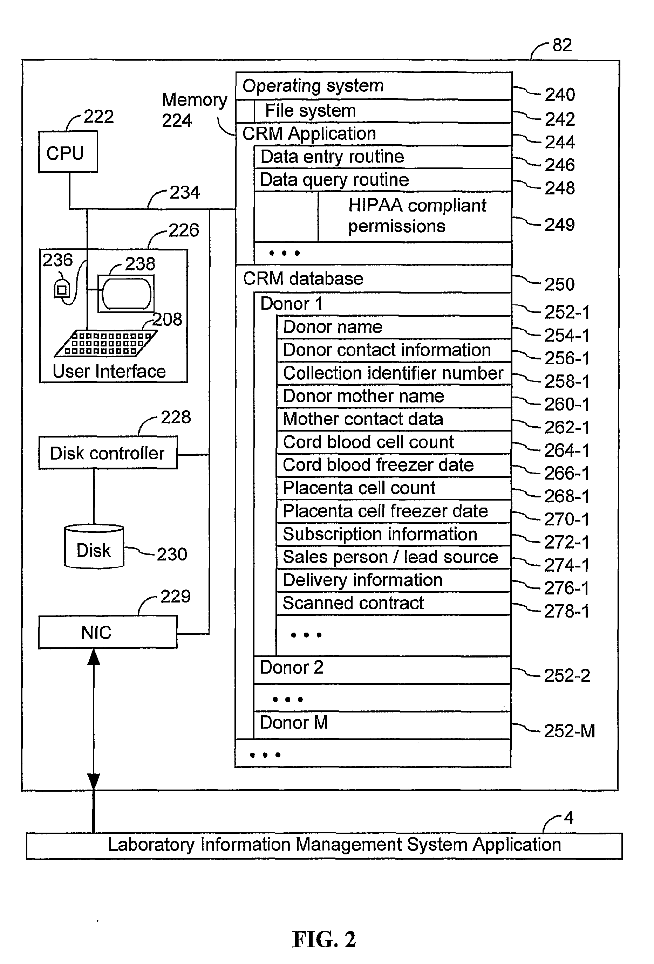

Figs 1 and 2 detail a system 10 that supports the functionality described above. System 10 preferably includes:
a computer system 4 for hosting a laboratory information management system application (Fig. 1); and
a computer system 82 for hosting a customer relationship management application.(Fig. 2).

Computer system 4 preferably includes:
- a central processing unit 22;
- a main non-volatile storage unit 30, for example a hard disk drive, for storing software and data, the storage unit 30 controlled by storage controller 28;
- a system memory 24, preferably high speed random-access memory (RAM), for storing system control programs, data, and application programs, comprising programs and data loaded from non-volatile storage unit 30; system memory 24 may also include read-only memory (ROM);
- a user interface 26, comprising one or more input devices (*e.g.,* keyboard 8, mouse 36) and a display 38 or other output device;
- a network interface card 29 for connecting to any wired or wireless communication network; and
- an internal bus 34 for interconnecting the aforementioned elements of the system.

Operation of computer 4 is controlled primarily by operating system 40, which is executed by central processing unit 22. Operating system 40 can be stored in system memory 24. In a typical implementation, system memory 24 includes:
- operating system 40;
- file system 42 for controlling access to the various files and data structures used by the present invention;
- a stem cell tracking module 44, also know as a laboratory information management system (LIMS) application, for receiving a stem cell characterization from a donor;
- a stem cell database 52 comprising information about a plurality of stem cell units; and
- a conversion interface 80 for facilitating the communication of information between stem cell tracking module 44 (LIMS application) and a customer relationship management application (CRM application).

Computer system 82 preferably includes:
- a central processing unit 222;
- a main non-volatile storage unit 230, for example a hard disk drive, for storing software and data, the storage unit 230 controlled by storage controller 228;
- a system memory 224, preferably high speed random-access memory (RAM), for storing system control programs, data, and application programs, comprising programs and data loaded from non-volatile storage unit 230; system memory 224 can also include read-only memory (ROM);
- a user interface 226, comprising one or more input devices (*e.g.,* keyboard 208, mouse 236, and/or touch screen display) and a display 238 or other output device;
- a network interface card 229 for connecting to any wired or wireless communication network; and
- an internal bus 234 for interconnecting the aforementioned elements of the system.

Operation of computer 82 is controlled primarily by operating system 240, which is executed by central processing unit 222. Operating system 240 can be stored in system memory 224. In a typical implementation, system memory 224 includes:
- operating system 240;
- file system 242 for controlling access to the various files and data structures used by the present invention;
- a customer relationship management application 244 for enrolling a donor; and
- a customer relationship management database 250 comprising information about a plurality of donors.

In a preferred embodiment of system 10, stem cell tracking module 44 and customer relationship management application 244 are stored on different computers (*e.g.,* computers 4 and 82). In other embodiments, system 10 comprises a single computer, with stem cell tracking module 44, stem cell database 52, CRM application 244 and CRM database 250 are all on the same computer. In fact, the present invention is not bound by the physical location of any of these constructs. As such, stem cell tracking module 44, stem cell database 52, CRM application 244, and CRM database 250 can each independently reside on the same or different computers and each construct can reside on one or more computers.

As illustrated in Figs. 1 and 2, system 10 includes one or more databases (*e.g*., database 52 and database 250). These database comprise any form of data storage system, comprising but not limited to a flat file, a relational database (SQL), and an on-line analytical processing (OLAP) database (MDX and/or variants thereof). In some specific embodiments, database 52 and/or database 250 is a hierarchical OLAP cube. In some specific embodiments, database 52 and/or database 250 comprises a star schema that is not stored as a cube but has dimension tables that define hierarchy. Still further, in some embodiments, database 52 and/or database 250 has hierarchy that is not explicitly broken out in the underlying database or database schema (*e.g.,* dimension tables are not hierarchically arranged). In some embodiments, there is only a single database that includes the illustrated functionality of both stem cell database 52 and CRM database 250. In typical embodiments, database 52 and/or database 250 is not hosted by respective computer 4 and 82. Rather, these database are accessed by these computers through a network interface. Section 5.8 describes exemplary architectures for the stem cell database 52 and/or CRM database 250.

It will be appreciated that many of the modules illustrated in Figs. 1 and 2 can be located on a remote computer. For example, some embodiments of the present application are web service-type implementations. In such embodiments, stem cell tracking module 44 and/or CRM application can reside on a client computer that is in communication with computer 4 and/or computer 82 via a network (not shown). In some embodiments, stem cell tracking module 44 and/or CRM application can be an interactive web page. In some embodiments, the databases and modules illustrated in Figs 1 and 2 are on a single computer and in other embodiments these databases and modules are found among several computers. Any arrangement is within the scope of the present invention so long as these components are addressable with respect to each other across a network or other electronic means. Thus, the present invention fully encompasses a broad array of computer systems.

Now that an overview of a system 10 in accordance with one embodiment of the present invention has been described, various advantageous methods in accordance with the present invention will now be disclosed in the following sections.

### 5.2. ENROLLING A DONOR AND COLLECTING A STEM CELL UNIT

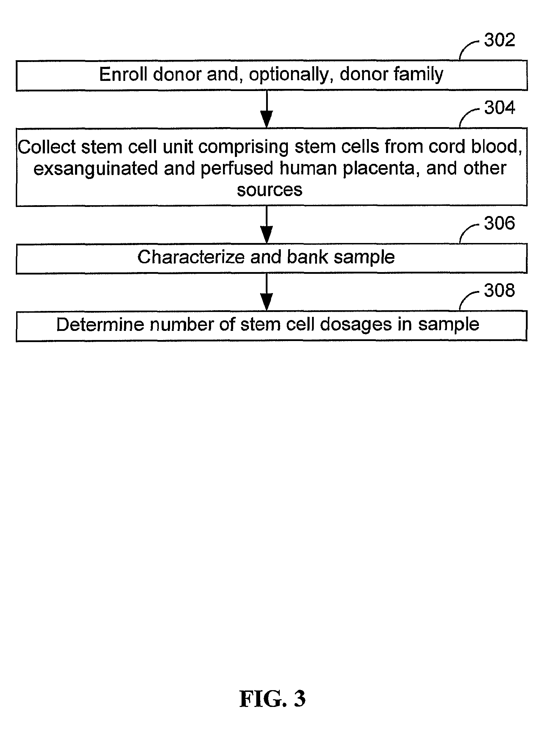

Fig. 3 illustrates a method for enrolling a donor and, optionally, the donor family in accordance with one embodiment of the present invention.

*Step 302.* In step 302, a donor is enrolled using CRM application 244 (Fig. 2). In some instances, such enrollment occurs before a child is born. Some embodiments of the present invention provide a private banking service in which a family enrolls with the stem cell bank and pays a fee for the collection and storage of the stem cells to be collected from their new-born.

Upon enrollment, a donor record 252 is created for the potential donor family in CRM database 250 using data entry routine 246 of the CRM application 244. Some of the information needed to complete the record 252 is provided at the time of enrollment, with other information, such as the sex of the donor infant may not yet be known upon enrollment.

At the time of enrollment, each potential donor is provided with a unique collection identifier number 258 that will become associated with their stem cell unit once it is collected. Upon enrollment, the donor family can elect to make the stem cell unit available to the public for searching and possible use. Such elections are stored in the subscription information 272 portion of the donor record. For instance, a notation that the donation is "available" can be set to "Yes" in the subscription information field and such a flag is otherwise set to "No" when the donation is a private donation for which the family of the donor have paid a fee.

Additional information that is stored in a record 252 may vary. However, in one embodiment of the present invention such information includes donor name 254, donor contact information 256 (*e.g.,* address, telephone number, *etc.),* as well as the name of the donor's mother 260 and her contact information. Record 252 may further include the name of the donor's father and his contact information. In some embodiments, record 252 further includes fields that allow for the tracking of the health of the donor, the donor's mother, and possibly the donor's father over the time that the stem cell unit from the donor is stored in a stem cell bank.

Record 252 is ultimately part of a comprehensive customer relationship management application 244. Accordingly, any type of referral information 274 that lead to securing the donation can be stored in record 252, such as the name of the sales person responsible for the sale. In addition, delivery information 276 is stored in record 252. Delivery information 276 includes, for example, the name of the hospital where the donation was made, the name of the physician that delivered the donor, and/or the contact information for this physician. Record 252 can optionally store images 278 of the contract that the donor's parents made with the stem cell bank for storing the stem cells.

*Step 304.* When the donor infant is born, the stem cell unit is collected, processed and banked. Preferably, the stem cell unit is stored in a cryogenic tank that can be accessed at a later time, as needed. In some embodiments, the stem cells are collected from not only cord blood, but other stem cell sources as well. Such additional sources include but are not limited to, for example, cells derived from bone marrow and cells obtained from the fetal placenta. In some embodiments, cord blood is collected using well known techniques and stem cells from the placenta are obtained using the techniques disclosed in Section 5.7, below. In some embodiments, donor is involved in a multiple birth. Thus, in some embodiments, there may be multiple placentae, such as in the case of a bi-chorial pregnancy that is bi-amniotic. The present invention fully encompasses such multiple birth situations.

In preferred embodiments of the present invention, stem cells are obtained from cord blood as well as from the fetal placenta that is procured, processed and from which stem cells are then removed for banking. In some embodiments, after stem cells have been obtained from the cord blood and certain tissues of the donor, they are cultured using stem cell expansion techniques.

Stem cell expansion techniques are disclosed in U.S. Patent No. 6,326,198 to Emerson et al.*,* entitled "Methods and compositions for the ex vivo replication of stem cells, for the optimization of hematopoietic progenitor cell cultures, and for increasing the metabolism, GM-CSF secretion and/or IL-6 secretion of human stromal cells," issued December 4, 2001; U.S. Patent No. 6,338,942 to Kraus et al.*,* entitled "Selective expansion of target cell populations," issued January 15, 2002; and U.S. Patent No. 6,335,195 to Rodgers et al.*,* entitled "Method for promoting hematopoietic and mesenchymal cell proliferation and differentiation," issued January 1, 2002, which are hereby incorporated by reference in their entireties.

Thus, in some embodiments, stem cells obtained from the donor are cultured in order to expand the population of stem cells. In other more preferred embodiments, however, stem cells collected from cord blood, exsanguinated human placenta post-partum, and other donor sources for use in accordance with any of the methods of the present invention are not expanded using such techniques. In fact, in preferred embodiments, such stem cells are substantially uncultured. This means that, while the stem cells can be stored for some period of time under conditions that allow the cells to survive, such storage conditions are chosen without any deliberate attempt to cause the cells to divide and, as a result, increase in number.

A stem cell unit can be collected from multiple sources, comprising both cord blood and exsanguinated post-partum placenta. The cord blood is a source of hematopoietic progenitor stem cells In one embodiment of the invention, such stem cells can be characterized by the presence of the cell surface markers such as but not limited to: CD10+, CD29+, CD34-, CD38-, CD44+, CD45-, CD54+, CD90+, SH2+, SH3+, SH4+, SSEA3-, SSEA4-, OCT-4+, and APC-p+. The presence or absence of such cell markers can be determined using techniques such as flow cytometry and immunocytochemistry In a preferred embodiment, such stem cells can be characterized by the presence of cell surface markers OCT-4+ and APC-p+. Such cells are as versatile (*e.g*., pluripotent) as human embryonic stem cells.

Embryonic-like stem cells originating from placenta have characteristics of embryonic stem cells but are not derived from the embryo. In other words, OCT-4+ and APC-p+ cells that are undifferentiated stem cells can be isolated from post-partum perfused placenta in addition to cord blood. Moreover, in some embodiments, a number of different pluripotent or multipotent stem cells can be isolated from the perfused placenta at different time points *e.g.,* CD34+/CD38+, CD34+/CD38-, and CD34-/CD38-hematopoietic cells. Thus, in some embodiments of the present invention, the stem cells in a stem cell unit are homogenous (e.g. all OCT-4+ and APC-p+) and in some embodiments, the stem cells in a stem cell unit are heterogeneous, with some OCT-4+ and APC-p+ cells combined with more committed cells.

Standard methods can be used to cyropreserve the stem cell units. In some instances, the stem cell units are stored in ten percent dimethyl sulfoxide when frozen. See, for example, Rubinstein et al., 1993, "Stored placental blood for unrelated bone marrow reconstitution" Blood 81, p. 1679-1690; Rubinstein et al. 1995, "Processing and cryopreservation of placental / umbilical cord blood for unrelated bone marrow reconstitution," Proc. Natl. Acad. Sci. USA 86, p. 3828-3832. In other instances, hydroxyl-ethyl starch sedimentation for volume reduction and red cell removal. See for example, Gluckman et al., 1997, "Use of Cord Blood Cells for Banking and Transplant," The Oncologist 2, pp. 340-343; and Rubinstein et al. 1995, "Processing and cryopreservation of placental/umbilical cord blood for unrelated bone marrow reconstitution," Proc Natl. Acad Sci USA 95, pp. 10112-10119.

*Step 306.* In step 306, the stem cells obtained in step 304 are characterized. In many instances, stem cells from at least two different origins of the donor are collected. For example, in a preferred embodiment, a first origin in the at least two different origins is cord blood of the donor and a second origin in the at least two origins is the placenta of the donor. Typically, the stem cells from different origins of the donor are characterized separately. The characterized information is stored in stem cell database 52 using the data entry routine 46 of stem cell tracking module 46. Stem cell database 52 includes a donation record 54 for each donation tracked by stem cell database 52. Each donation record 54 (Fig. 1) includes a unique collection identifier number 58 (Fig. 1) that corresponds to the collection identifier number 258 (Fig. 2) assigned to a donor by CRM application 244 (Fig. 2). In some embodiments, each donation record 54 (Fig. 1) includes a maternal identifier number 62 that uniquely identifies the donor's mother. Typically, maternal identifier number 62 is assigned by CRM application 24.

Fig. 1 illustrates the case in which stem cells are collected from two different origins, cord blood and the placenta. As such, there is a cord blood component 66 that stores the characterization of the stem cells from the donor's cord blood and there is a placenta component 72 that stores the characterization of the stem cells from the donor's placenta. However, the invention is not limited to such an embodiment. In other embodiments, each record 54 includes additional components to store the characterization of stem cells from other sites of origin of the donor, such as bone marrow.

In one embodiment of the invention, stem cells are obtained from cord blood and the placenta and each donation record 54 includes a cord blood component 66 for storing a characterization of the donor cord blood and a placenta component 74 for storing a characterization of the placenta fluid. In this embodiment of the invention, the cord blood characterization includes the results of various assays 68, comprising a sterility test and the results of an infectious disease test 70. The sterility test is run to ensure that the cord blood is sterile while the infectious disease test 70 determines whether the cord blood has an infectious disease such as human immunodeficiency virus, Epstein-Barr virus, hepatitis B, hepatitis C, syphilis, or cytomegalovirus. Tests for infectious agents are described in Rubinstein, 1994, Blood Cells 20, p. 587. The test for cytomegalovirus can be performed, for example, using the CMV-M EIA diagnostic kit, Abbott Laboratories, North Chicago, Illinois. The cord blood characterization further includes a CD34 marker test. The cord blood characterization further includes an HLA blood type test. HLA blood type plays a major role in determining whether blood will be accepted as self (histocompatible) or rejected as foreign (histoincompatible). More information on HLA type and methods for determining HLA type are found in United States Patent 6,670,124 B1, which is hereby incorporated by reference in its entirety. Finally, the cord blood component 66 includes a cord blood cell count 72. The placenta component 74 of donor record 54 also includes assay test results 76 and a placenta stem cell count 78. The assay tests performed on the placenta stem cell unit includes an endotoxin test, a sterility test, and a CD34 marker test for stem cells.

In some embodiments, characterizing step 306 comprises performing a nucleated cell count on the stem cell unit, performing a leukocyte count on the stem cell unit, performing a mononuclear cell count on the stem cell unit, performing a CD34-positive cell count on the stem cell unit, performing a CD3-positive cell count on the stem cell unit, performing a colony-forming cell assay on the stem cell unit, performing an infectious disease assay on the stem cell unit, or determining an HLA blood type of the stem cell unit.

In some embodiments, the stem cell unit comprises a first class of stem cells and a second class of stem cells. The first class of stem cells originates from cord blood of the donor and the stem cells in the second class of stem cell transplant units originates from the placenta of the donor. In such embodiments, characterizing step 306 comprises separately characterizing the first class of stem cells and the second class of stem cells by performing any or all of the assays described above.

*Step 308.* The methods used in step 304 to collect stem cells from single donor, in many instances, will result in more stem cells than are needed to treat a patient. If the number of stem cells needed to treat a patient is termed a "stem cell transplant unit," the stem cell transplant unit will be some fraction of the total number of stem cells collected from a single donor. In step 308, the number of stem cell transplant units in the unit collected in step 304 is estimated. This estimate can only be approximate because the number of stem cells needed to treat a patient is dependent on factors that cannot be quantified at the time estimate 308 is made. These factors include the weight of the patient, whether or not the patient is a sibling of the donor (as opposed to bearing no relationship with the donor), and the percentage of stem cells that will be lost upon thawing the stem cell unit provided by the donor. Typically, cord blood obtained by conventional means contains sufficient numbers of stem cells for engraftment in most recipients weighing less than 50 kilograms (about 110 pounds).

In typical embodiments, the stem cells from each unique source of the donor are kept separate from each other. Thus, in typical embodiments of the present invention, a stem cell unit from a single donor comprises stem cells originating from the placenta, stem cells originating from the cord blood, and possibly stem cells originating from other sources. The stem cells from each of these different sources is referred to as a stem cell unit. In other words, a stem cell unit refers to a population comprising stem cells, from a single donor that have been arrived from a single source such as, for example, an exsanguinated placenta, cord blood, fetal tissue, bone marrow, or any other tissue or other organ from which stem cells may be derived. Thus the stem cell unit obtained in step 304 may contain multiple stem cell units and each stem cell unit may include multiple stem cell transplant units.

A stem cell transplant unit refers to a population comprising stem cells, from a single donor, that have been derived from any single such source. A placental stem cell transplant unit refers to a population comprising stem cells, from a single donor, that have been derived from a placenta (or multiple placentae in the case of a multiple birth). Common to stem cell transplant units and placental stem cell transplant units is that they each comprise a therapeutically effective amount of stem cells.

The number of nucleated cells in a stem cell unit varies. Furthermore, the number of nucleated cells in a stem cell unit may be less after freezing/thawing. Consequently, in reviewing stem cell transplantation data, it is instructive to note whether nucleated cell count was measured before or after thawing the unit. For example, in Sanz *et al.,* the median proportion of nucleated cells lost during thawing was thirty percent. See Sanz et al., 2001, "Standardized, unrelated donor cord blood transplantation in adults with hematologic malignancies," Blood 98, p. 2332.

In some embodiments, the number of nucleated stem cells in a stem cell transplant unit (or a placental stem cell transplant unit) is a function of the weight of a patient and the number of cells per kilogram of patient weight that are to be delivered to such a patient. In other embodiments, the number of nucleated stem cells in a stem cell transplant unit (or a placental stem cell transplant unit) is not a function of the weight of the patient. Rather, in such embodiments, the weight of the patient or other characteristics of the patient are used to determine how many stem cell transplant units (or placental stem cell transplant units) are to be delivered to the patient. Examples of such embodiments will now be described. Throughout these examples, it will be appreciate that, in accordance with the advantages of the present invention, nucleated cell counts refers to cell populations that have not undergone substantial clonal expansion.

*Embodiments where the number of nucleated cells in a stem cell transplant unit (or placental stem cell transplant unit) is a function of patient body weight.* In embodiments where the number of nucleated cells in a stem cell transplant unit (or a placental stem cell transplant unit) is a function of patient weight, a determination is made as to how many nucleated cells per kilogram of patient weight is needed. For example, in some embodiments, an assumption is made that a patient will need about 10 million (15 x 10⁶) nucleated cells per kilogram of body weight. In such embodiments, the number of nucleated cells in a stem cell transplant unit is determined on a patient specific basis as a function of patient body weight as follows:

| Patient Body Weight | Number of nucleated cells in a stem cell transplant unit |
|---|---|
| 10 kilograms | 150 x 10⁶ |
| 20 kilograms | 300 x 10⁶ |
| 30 kilograms | 450 x 10⁶ |
| 40 kilograms | 600 x 10⁶ |
| 50 kilograms | 750 x 10⁶ |
| 60 kilograms | 900 x 10⁶ |
| 70 kilograms | 1050 x 10⁶ |
| 80 kilograms | 1200 x 10⁶ |
| 90 kilograms | 1350 x 10⁶ |
| 100 kilograms | 1500 x 10⁶ |

The number of nucleated cells in a stem cell transplant unit for patients having a body weight other than that listed in the table above is computed as a linear interpolation of the values presented in the table. As another example, in some embodiments, an assumption is made that a patient will need about 25 million (25 x 10⁶) nucleated cells per kilogram of body weight. In such embodiments, the number of nucleated cells in a stem cell transplant unit is determined on a patient specific basis as a function of patient body weight as follows:

| Patient Body Weight | Number of nucleated cells in a stem cell transplant unit |
|---|---|
| 10 kilograms | 250 x 10⁶ |
| 20 kilograms | 500 x 10⁶ |
| 30 kilograms | 750 x 10⁶ |
| 40 kilograms | 1000 x 10⁶ |
| 50 kilograms | 1250 x 10⁶ |
| 60 kilograms | 1500 x 10⁶ |
| 70 kilograms | 1750 x 10⁶ |
| 80 kilograms | 2000 x 10⁶ |
| 90 kilograms | 2250 x 10⁶ |
| 100 kilograms | 2500 x 10⁶ |

The number of nucleated cells in a stem cell transplant unit for patients having a body weight other than that listed in the table above is computed as a linear interpolation of the values presented in the table. In a similar manner, the assumption is made that a patient will need between 15 x 10⁶ and 25 x 10⁶ nucleated cells per kilogram of patient body weight, between 25 x 10⁶ and 50 x 10⁶ nucleated cells per kilogram of patient body weight, more than 50 x 10⁶ nucleated cells per kilogram of patient body weight, or less than 15 x 10⁶ nucleated cells per kilogram of patient body weight. In such embodiments, the number of nucleated cells in a given stem cell transplant unit (or placental stem cell transplant unit) is determined using table similar to those presented above, or interpolation of values in such tables.

In some embodiments, there are least 0.4 x 10⁷ nucleated cells per kilogram of patient where the nucleated cells are obtained using the methods described in step 304 and where cell measurement is made prior to freezing or thawing the source stem cell unit. In other embodiments of the present invention, a stem cell transplant unit is at least 1.0 x 10⁷ nucleated cells per kilogram of patient, at least 1.5 x 10⁷ nucleated cells per kilogram of patient, at least 2.0 x 10⁷ nucleated cells per kilogram of patient, at least 2.5 x 10⁷ nucleated cells per kilogram of patient, at least 3.0 x 10⁷ nucleated cells per kilogram of patient, at least 3.5 x 10⁷ nucleated cells per kilogram of patient, at least 4.0 x 10⁷ nucleated cells per kilogram of patient, at least 4.5 x 10⁷ nucleated cells per kilogram of patient, or at least 5.0 x 10⁷ nucleated cells per kilogram of patient where the nucleated cells are obtained using the methods described in step 304 and where cell measurement is made prior to freezing or thawing the transplant unit. In some embodiments of the present invention, a stem cell transplant unit is between 0.4 x 10⁷ and 25 x 10⁷ nucleated cells per kilogram of patient, between 0.8 x 10⁷ and 12 x 10⁷ nucleated cells per kilogram of patient, between 1 x 10⁷ and 10 x 10⁷ nucleated cells per kilogram of patient, or between 1 x 10⁷ and 8 x 10⁷ nucleated cells per kilogram of patient where the nucleated cells are obtained using the methods described in step 304 and where cell measurement is and/or characterization is made prior to freezing or thawing the transplant unit.

In some embodiments of the present invention, a stem cell transplant unit is at least 0.4 x 10⁷ nucleated cells per kilogram of patient where the nucleated cells are obtained using the methods described in step 304 and where cell measurement is made after freezing and thawing the source stem cell unit. In other embodiments of the present invention, a stem cell transplant unit is at least 1.0 x 10⁷ nucleated cells per kilogram of patient, at least 1.5 x 10⁷ nucleated cells per kilogram of patient, at least 2.0 x 10⁷ nucleated cells per kilogram of patient, at least 2.5 x 10⁷ nucleated cells per kilogram of patient, at least 3.0 x 10⁷ nucleated cells per kilogram of patient, at least 3.5 x 10⁷ nucleated cells per kilogram of patient, at least 4.0 x 10⁷ nucleated cells per kilogram of patient, at least 4.5 x 10⁷ nucleated cells per kilogram of patient, or at least 5.0 x 10⁷ nucleated cells per kilogram of patient where the nucleated cells are obtained using the methods described in step 304 and where cell measurement is made after freezing and thawing the stem cell transplant unit. In some embodiments of the present invention, a stem cell transplant unit is between 0.4 x 10⁷ and 25 x 10⁷ nucleated cells per kilogram of patient, between 0.8 x 10⁷ and 12 x 10⁷ nucleated cells per kilogram of patient, between 1 x 10⁷ and 10 x 10⁷ nucleated cells per kilogram of patient, or between 1 x 10⁷ and 8 x 10⁷ nucleated cells per kilogram of patient where the nucleated cells are obtained using the methods described in step 304 and where cell measurement is made after freezing and thawing the transplant unit.

In some embodiments of the present invention, a separate determination is made of the number of stem cell transplant units from each different origin of the donor. Thus, in the embodiment illustrated in Fig. 1, the number of stem cell transplant units in the stem cell unit originating from cord blood is distinguished from the number of stem cell transplant units in the stem cell unit originating from the placenta. In Fig. 1, the stem cell transplant unit estimates are stored as element 64 in the record 54 associated with the donation.

In some embodiments of the present invention, the number of stem cell transplant units present in a stem cell donation is estimated based on an average patient weight of 20 kilograms, 25 kilograms, or 30 kilograms and a stem cell,transplant unit of 1.0 x 10⁷ or 2.0 x 10⁷ nucleated cells per kilogram of patient. In this way, standardized units of stem cell transplant units can be defined so that estimates of the number of stem cell transplant units in a stem cell donation can be made.

In some embodiments of the present invention, a placental stem cell transplant unit is at least 0.4 x 10⁷ nucleated cells per kilogram of patient where the nucleated cells are obtained using the methods described in step 304 and where cell measurement is made prior to freezing or thawing the source placental stem cell transplant unit. In other embodiments of the present invention, a placental stem cell transplant unit is at least 1.0 x 10⁷ nucleated cells per kilogram of patient, at least 1.5 x 10⁷ nucleated cells per kilogram of patient, at least 2.0 x 10⁷ nucleated cells per kilogram of patient, at least 2.5 x 10⁷ nucleated cells per kilogram of patient, at least 3.0 x 10⁷ nucleated cells per kilogram of patient, at least 3.5 x 10⁷ nucleated cells per kilogram of patient, at least 4.0 x 10⁷ nucleated cells per kilogram of patient, at least 4.5 x 10⁷ nucleated cells per kilogram of patient, or at least 5.0 x 10⁷ nucleated cells per kilogram of patient where the nucleated cells are obtained using the methods described in step 304 and where cell measurement is made prior to freezing or thawing the unit. In some embodiments of the present invention, a placental stem cell transplant unit is between 0.4 x 10⁶ and 100 x 10⁶ nucleated cells per kilogram of patient, between 0.8 x 10⁶ and 50 x 10⁶ nucleated cells per kilogram of patient, between 15 x 10⁶ and 35 x 10⁶ nucleated cells per kilogram of patient, or between 17 x 10⁶ and 30 x 10⁶ nucleated cells per kilogram of patient where the nucleated cells are obtained using the methods described in step 304 and where cell measurement is made prior to freezing or thawing the unit.

In some embodiments of the present invention, a placental stem cell transplant unit is at least 0.4 x 10⁶ nucleated cells per kilogram of patient where the nucleated cells are obtained using the methods described in step 304 and where cell measurement is made after freezing and thawing the source stem cell unit. In other embodiments of the present invention, a placental stem cell transplant unit is at least 1.0 x 10⁶ nucleated cells per kilogram of patient, at least 5 x 10⁶ nucleated cells per kilogram of patient, at least 10 x 10⁶ nucleated cells per kilogram of patient, at least 20 x 10⁶ nucleated cells per kilogram of patient, at least 30 x 10⁶ nucleated cells per kilogram of patient, at least 35 x 10⁶ nucleated cells per kilogram of patient, at least 40 x 10⁶ nucleated cells per kilogram of patient, at least 45 x 10⁶ nucleated cells per kilogram of patient, or at least 50 x 10⁶ nucleated cells per kilogram of patient where the nucleated cells are obtained using the methods described in step 304 and where cell measurement is made after freezing and thawing the unit. In some embodiments of the present invention, a placental stem cell transplant unit is between 0.4 x 10⁶ and 100 x 10⁶ nucleated cells per kilogram of patient, between 1 x 10⁶ and 50 x 10⁶ nucleated cells per kilogram of patient, between 5 x 10⁶ and 40 x 10⁶ nucleated cells per kilogram of patient, or between 20 x 10⁶ and 30 x 10⁶ nucleated cells per kilogram of patient where the nucleated cells are obtained using the methods described in step 304 and where cell measurement is made after freezing and thawing the unit.

*Embodiments where the number of nucleated cells in a stem cell transplant unit (or placental stem cell transplant unit) is not a function of patient body weight.* In some embodiments of the present invention, a stem cell transplant unit and/or placental cell transplant unit has at predetermined number of nucleated cells regardless of the intended use of the unit. In some instances in accordance with such embodiments, there are between 150 x 10⁶ and 10000 x 10⁶ nucleated cells, between 300 x 10⁶ and 5000 x 10⁶ nucleated cells, or between 500 x 10⁶ and 3000 x 10⁶ nucleated cells. Each such stem cell transplant unit need not be equally divided units. In some embodiments, a stem cell transplant unit is defined in terms of a number of nucleated cells. In some embodiments, a stem cell transplant unit is between 25 and 50 x 10⁷ nucleated cells, between 50 and 100 x 10⁷ nucleated cells, between 100 and 150 x 10⁷ nucleated cells, between 150 and 200 x 10⁷ nucleated cells, between 200 and 250 x 10⁷ nucleated cells, between 250 and 300 x 10⁷ nucleated cells, or between 300 and 500 x 10⁷ nucleated cells. In some embodiments, the stem cell transplant unit is defined in terms of the number of nucleated cells in the unit before it is frozen and thawed whereas in other embodiments the stem cell transplant unit is defined in terms of the number of nucleated cells in the same after it has been frozen and thawed.

Although stem cell transplant units have been defined herein in terms of the number of nucleated cells in a unit, the present invention is not limited to such a metric. Alternative metrics can be used to define a stem cell transplant unit such as the number of CD34+ cells collected or thawed, the number of colony forming cells collected or thawed, the number of granulocyte-macrophage colony forming cells collected or thawed, the number of CD3+ cells collected or thawed, the number of burst forming unit-erythroid cells collected or thawed, or the number of colony forming unit-granulocyte erythroid monocyte macrophage cells collected or thawed. See Gluckman et al., 1997, The Oncologist 2, 340; Migliaccio et al., 2000, Blood 96, 2717; and Thomson et al., 2000, Blood 96 2703 for more information on how such cells measurements can be made.

In some embodiments of the present invention, a placental stem cell transplant unit is defined in terms of a number of nucleated cells. In some embodiments, a placental stem cell transplant unit is between 25 and 50 x 10⁷ nucleated cells, between 50 and 100 x 10⁷ nucleated cells, between 100 and 150 x 10⁷ nucleated cells, between 150 and 200 x 10⁷ nucleated cells, between 200 and 250 x 10⁷ nucleated cells, between 250 and 300 x 10⁷ nucleated cells, or between 300 and 500 x 10⁷ nucleated cells. In some embodiments, the placental stem cell transplant unit is defined in terms of the number of nucleated cells in the unit before it is frozen and thawed whereas in other embodiments the placental stem cell transplant unit is defined in terms of the number of nucleated cells in the same after it has been frozen and thawed.

Although placental stem cell transplant units have been defined herein in terms of the number of nucleated cells in a placental stem cell transplant unit, the present invention is not limited to such a metric. Alternative metrics can be used to define a placental stem cell transplant unit such as the number of CD34+ cells collected or thawed, the number of colony forming cells collected or thawed, the number of granulocyte-macrophage colony forming cells collected or thawed, the number of CD3+ cells collected or thawed, the number of burst forming unit-erythroid cells collected or thawed, or the number of colony forming unit-granulocyte erythroid monocyte macrophage cells collected or thawed. See Gluckman et al., 1997, The Oncologist 2, 340; Migliaccio et al., 2000, Blood 96, 2717; and Thomson et al., 2000, Blood 96 2703 for more information on how such cells measurements can be made.

### 5.3. RECEIVING A QUERY FOR STEM CELLS AND DISBURSING ONE OR MORE STEM CELL TRANSPLANT UNITS WHEN THERE IS A MATCH BETWEEN DONOR AND PATIENT

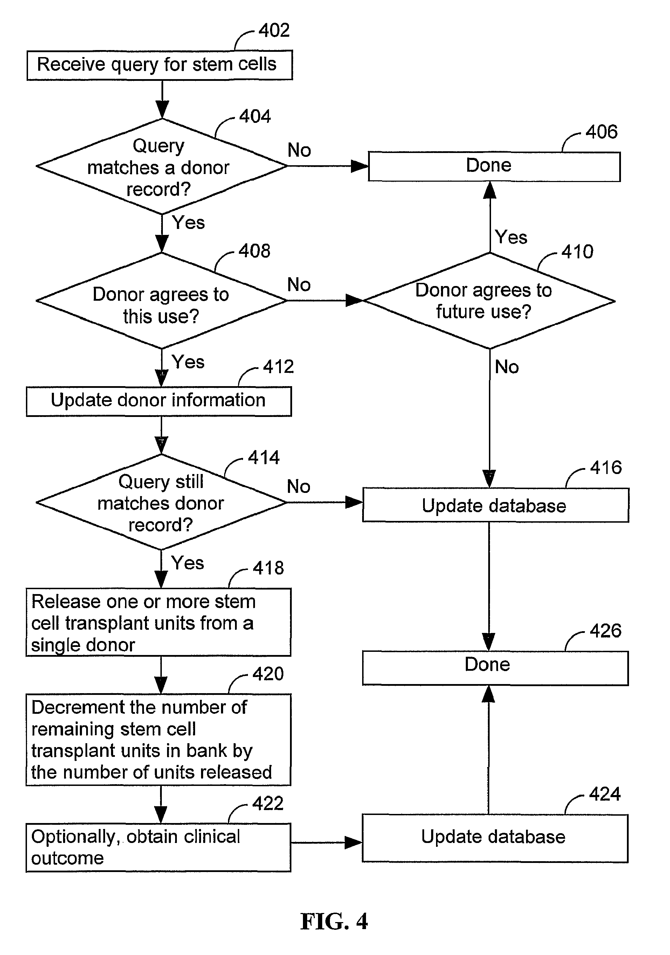

Fig. 4 illustrates a method for receiving a query for stem cells and for disbursing one or more stem cell transplant units when there is a match between donor and patient. Referring to step 402 of Fig. 4, when a donor is sought for a patient, data query routine 48 of stem cell tracking module 44 searches stem cell database 52 for a donor matching the party. In some embodiments, the patient has a disease identified in Section 5.6 and the process illustrated in Fig. 4 is designed to cure or alleviate this disease. The search uses an appropriate matching algorithm based on the data in records 54. In typical embodiments, the search attempts to find a donation in database 52 that has the same HLA type as the patient. If no matching donor record 54 is found (404-No), the processing is complete (406). On the other hand, if a matching donor record is found (404-Yes), then it is ascertained whether or not the donor agrees to donate one or more stem cell transplant units to the patient (408). In typical embodiments, step 408 comprises ascertaining whether or not the matching stem cell unit is public or private. The unit is private when the donor has paid to preserve the unit for use by the donor's family and the unit is public when the donor has not paid to preserve the unit. Information on whether a unit is public or private is stored in the public / private indicator 60 of each record 54.

If the donor does not agree to this use (408-No), because the unit is private for example, then it is optionally ascertained whether or not the donor will agree to any future uses of this unit (410). If the donor might agree to future uses of the unit (410-Yes) then processing is complete (406), otherwise (410-No) database 52 is optionally updated (416) to indicate that this unit is not available. That is, field 60 of the matching donor's record 52 is set to "NO" to indicate unavailability of this donor's unit. This scenario can occur when a donor decides that a unit should no longer be available and commits to paying a fee for the unit. In some embodiments, a donor that does not pay a fee to keep a unit private does not have the option to deny provision of the unit to a patient with matching characteristics. In some embodiments, a donor is nor provided with an option to convert his donation to private once the donor has enrolled as public.

If the donor does agree to the use of the unit, or if the unit is marked "public", (408-Yes), then, if possible, more information is optionally obtained from or about the donor (412). This information could include information that was not available or ascertainable at the time the unit was provided. For example, it would be desirable to know whether the donor's mother or the donor became HIV positive shortly after birth of the donor or whether the donor developed leukemia. Such adverse indications could be used as a basis for rejecting the unit.

If the donor record is still suitable after the optional donor update information derived in step 412 (414-Yes), process control passes to step 418. Otherwise (414-No), process control passes to step 416 where stem cell database 52 is updated to reflect that adverse indications have arisen in the donor and the donation record 54 is withdrawn from database 52.

If the donated unit is acceptable and available (414-Yes), then one or more stem cell transplant units and/or one or more placental stem cell transplant units are released for use by the patient (418). As detailed in the discussion of step 308 in Section 5.2, the number of stem cell transplant units released to the patient is a function of how a stem cell transplant unit is quantified, the weight of the patient and, optionally, whether the patient and donor are related to each other. For the sake of illustration, a stem cell transplant unit is defined as the number of stem cells needed to provide a 25kg patient with 1.5 x 10⁷ nucleated cells per kilogram of patient mass (37.5 x 10⁷ nucleated cells). Consider the case in which the placenta component of the matching stem cell donation comprises 75 x 10⁷ nucleated cells. In this case, the placenta component of the matching stem cell donation comprises two stem cell transplant units. Completion of step 418, then, requires a determination of the number of stem cells needed for the patient. In preferred embodiments, the physician responsible for engrafting the stem cells into the patient will make this decision based on at least two factors (i) a desired number of nucleated cells per kilogram of patient mass and (ii) the mass of the patient. For example, if the doctor desires 1.5 x 10⁷ nucleated cells per kilogram of patient mass and the patient weighed 1 kilogram, then exactly one stem cell transplant unit would be released in step 418.

In step 420, the number of remaining stem cell transplant units is decremented from field 64 of the matching record 54 in stem cell database 52 to reflect the number of stem cell transplant units assigned to the matching patient. In step 422 the clinical outcome 422 of the patient graft is optionally obtained. Such information can be used to determine the value of the remaining stem cell transplant units in the original donation. For example, if the graft was successful, this would indicated that the remaining stem cell transplant units have significant medical value. On the other hand, if the risk factors for the patient were very low and the graft was still not successful, then this would suggest that the remaining stem cells may not be well preserved. Still other useful clinical outcome information includes a determination of whether any diseases that may have been caused by the blood, such as leukemia, arose in the patient. Such indications would be grounds for immediately removing all remaining stem cell transplant units from database 52. In step 424, stem cell database 52 is updated to reflect the number of stem cell transplant units in the matching donation remain in the database and, optionally to reflect clinical outcome of the patient. In step 426 the process is completed.

### 5.4. ENROLLING A DONOR, COLLECTING A STEM CELL UNIT AND PRESERVING DONOR ANONYMITY WHILE MAINTAINING CONTROL OVER ACCESS TO MEDICAL HISTORY AND TESTING OF THE STEM CELL UNIT

In addition to providing registries in which individual stem cell donations comprise multiple doses, the present invention provides methods for maintaining client anonymity. Referring once again to Figs 1 and Fig. 2, this is accomplished in one embodiment of the present invention by using a system that has two applications, a customer relationship management (CRM) application 244 and a laboratory information management system (LIMS) application (stem cell tracking module 44). CRM application 244, in conjunction with CRM database 250, maintains information about a donor, comprising the name of the donor 254, donor contact information 256, the name of the donor's parents (*e.g.,* donor mother name 260), and contact information of the donor's parents (*e.g.,* mother contact data 262). CRM application 244, in conjunction with CRM database 250, further stores a collection identifier number 258 that is uniquely associated with a donor. CRM application 244 and CRM database 250, however, do not store stem cell characterization information, such as infectious disease results and HLA blood type. Rather, the LIMS application (stem cell tracking module 44 together with stem cell database 52) which is isolated from CRM application 244 either through electronic (password / permission) or physical (separate network / building) means, maintains the stem cell characterization information. The LIMS application does not track patient names and cannot access the patient's names. Rather, donations are tracked by collection identifier number 58 (Fig. 1) which is equivalent to the collection identifier number 258 of Fig. 2. In this way, lab workers entering sensitive assay results into the LIMS application cannot learn the identity of the donors. Further, administrators of CRM application 244 cannot access lab results on the LIMS application unless they have special privileges. The maintenance of donor anonymity in this way has significant marketing advantages over known blood banking techniques.

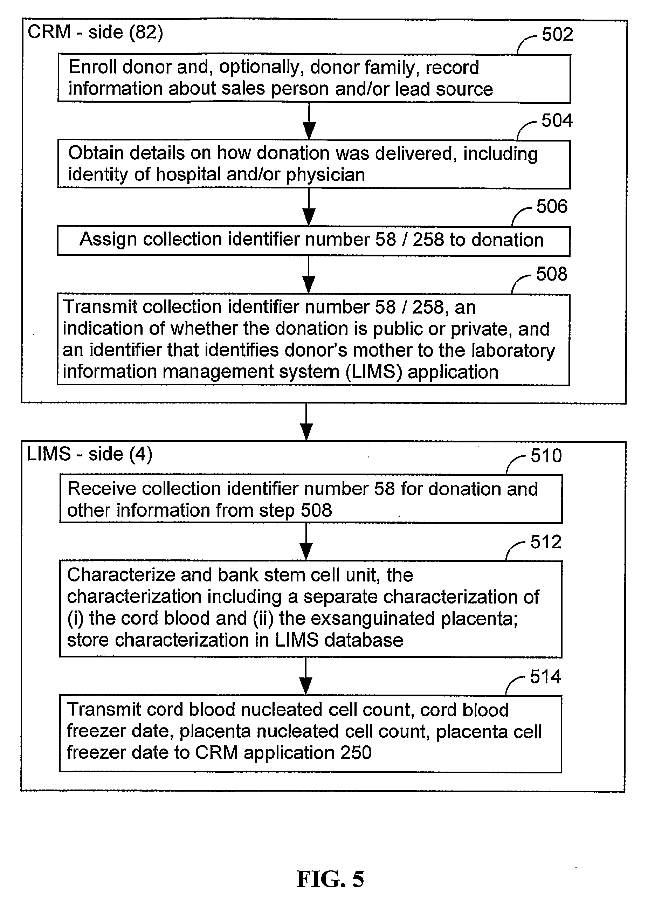

Fig. 5 illustrates a method for maintaining client anonymity in accordance with this aspect of the invention.

*Step 502.* In step 502, a donor is enrolled by data entry routine 244 of CRM application 244 (Fig. 2) by creating a donor record 252 for the donor in CRM database 250. The donor may pay a fee and enroll as a private donor so that the stem cell donation is not given to a patient without the donor's consent. Alternatively, the patient can pay no fee, in which case the unit is given to the first patient in need of the unit that has matching characteristics. In step 502, additional information about the origination of the donation, such as the sales person that is credited with procuring a private donation or the lead source that lead to the procurement of a private donation is stored in fields 274 of the donor record 252. Details on whether a donor paid a fee (private donor) or did not pay a fee (public donor) are store in subscription information field 272.

*Step 504.* In step 502, pertinent details on how the donation was obtained, comprising the identity of the hospital where the donation was made and/or the attending physician are obtained and stored as delivery information 276 of the donor record 252 uniquely associated with the donor in CRM database 250 (Fig. 2).

*Step 506.* In step 506, a collection identifier number 258 is assigned to the donation. The collection identifier number 258 can have any format so long as it is uniquely identifies the donor. The collection identifier number 258 is stored in the donor record 252 of the donor in CRM database 250. In some embodiments, collection identifier number 252 is simply the record id of the donor record 252 association with the donor. The collection identifier numbers 258 of CRM database 250 are equivalent to, or have a 1:1 correspondence with, the collection identifier numbers 58 of stem cell database 52. In addition to the collection identifier number 258, an anonymous identifier for the donor's mother is generated and stored in CRM database 250.

*Step 508.* In step 508, CRM application 244 transmits certain information to the stem cell tracking module 44 (LIMS application) for storage in stem cell database 52. In some embodiments, this information includes the collection identifier number 258 (58), an optional indication of whether the donation is public or private, and an identifier that identifies the donor's mother.

*Step 510.* Steps 510 through 514 represent activity that occurs in the LIMS application (stem cell tracking module 44 together with stem cell database 52). In step 510, the information transmitted by CRM application 244, comprising the collection identifier number 58 (25), public/private indicia, and the mother identification number, are received and processed by data entry routine 46 of stem cell tracking module 44 for inclusion in a donation record 54 of stem cell database in computer 4 (Fig. 1). Thus, a direct result of step 510 is the creation of a donation record 52 in stem cell database 52 that corresponds to a donor record 252 in CRM database 250. However, unlike the donor record 252 in CRM database 250, the donation record 54 in stem cell database 52 does not identify the donor. All there is in donation record 54 is a collection identifier number 58. Thus, donor anonymity is preserved in the LIMS application (Fig. 1).

*Step 512.* In step 512 the donation is characterized and the results of this characterization are stored in the donation record 54 that corresponds to the donation. In preferred embodiments, the stem cell donation comprises a cord blood component and a exsanguinated placenta component. However, the invention is not limited to such donations and, in fact, the stem cell donation can be a cord blood unit obtained using conventional means. In one embodiment, characterization of the stem cell donation is as described in step 306 of Section 5.2. In particular, the HLA blood type of at least one portion of the stem cell donation is determined, and the stem cell donation is tested for infectious diseases such as HIV and hepatitis. The characterization further includes a nucleated cell count or other form of count that is intended to determine the viability of the stem cell unit. Additional data that can be stored in step 512 is the process record 56 for the stem cell donation. Process record 56 includes information on how the blood cell donation was physically handled (*e.g.,* when the unit was delivered to the lab).

*Step 514.* Step 514 completes the data entry process. In this step, a limited amount of nonsensitive information is returned to the CRM application. For instance, in embodiments where separate placenta and cord blood units are stored, step 514 comprises transmitting the cord blood nucleated cell count (or other indicia of stem cell unit viability), cord blood freezer date, placenta nucleated cell count (or other indicia of stem cell unit viability), and placenta cell freezer date. Such information is stored in fields 264 through 270 of the corresponding donor record 252 in CRM database 250.

One specific embodiment of the present invention provides a computer program product for use in conjunction with a computer system. The computer program product comprises a computer readable storage medium and a computer program mechanism embedded therein. The computer program mechanism comprises a customer relationship management database 250 comprising information about a plurality of donors and a customer relationship management application 244 (Fig. 2) that includes (i) instructions for enrolling a donor in the customer relationship management database 246, (ii) instructions for uniquely associating a collection identifier number to the donor, and (iii) instructions for transmitting the collection identifier number to a stem cell tracking module. In this embodiment, the computer program mechanism further comprises a stem cell database 52 comprising information about a plurality of stem cell units and a stem cell tracking module 44. Stem cell tracking module 44 includes a data entry routine 46 that comprises (i) instructions for receiving a collection identifier number 58 / 258 from customer relationship management application 244 and (ii) instructions for receiving a characterization of a stem cell unit from the donor. The characterization includes a characterization of stem cells from at least two different origins of the donor. In some embodiments, customer relationship management database 250 and stem cell database 52 are on two different computers and an access privilege 249 is required to access stem cell database 52 from CRM application 244. In some embodiments, stem cell database 52 is a relational database, an on-line analytical processing database, or a hierarchical on-line analytical processing data cube and the information about the plurality of stem cell units can be searched for a stem cell unit having characteristics that match a query search.

### 5.5. RECEIVING A QUERY FOR STEM CELLS AND DISBURSING STEM CELLS WHILE PRESERVING DONOR ANONYMITY

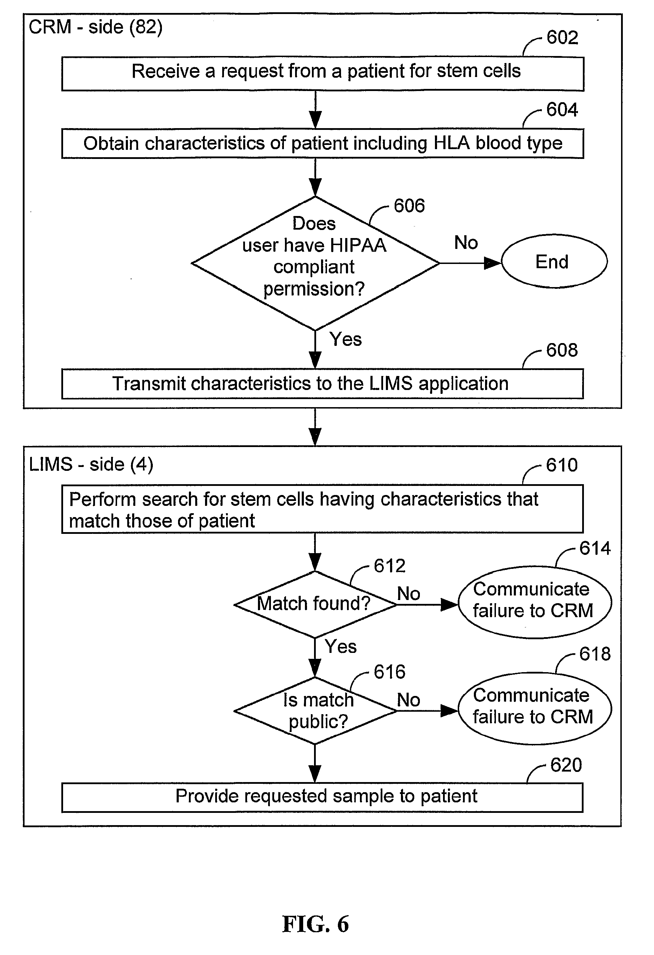

Section 5.4 describes a method for building a stem cell registry that isolates laboratory results from a customer relationship management application 244 thereby maintaining donor anonymity. Referring to Fig. 6, a method is disclosed for receiving a request for stem cells and searching a stem cell bank for matching stem cell units. Steps 602 through 608 take place on the CRM side (Fig. 2) of system 10 whereas steps 610 through 620 take place on the LIMS side (Fig. 1) of system 10.

*Steps 602 and 604.* In steps 602 and 604, a request is received by a patient for stem cells. The request is serviced by data query routine 248 of CRM application 244 (Fig. 2). The request will include sufficient characterization of the patient in order to obtain a suitable donor. For example, the characterization may include the HLA type of the patient, gender, age, and/or any other type of characterization used in the art to find a suitable donor.

*Step 606.* Before CRM database 250 can be searched for suitable donors (or at least before the results from a query of database 250 can be reviewed) a determination must be made to ensure that the query of the database 250 is authorized. In one embodiment, step 6060 is implemented as a set Health Insurance Portability and Accountability Act of 1996 (HIPAA) compliant permissions 249. If the user has HIPAA compliant authorization (606-Yes), the process continues. Otherwise (606-No), it ends.

*Step 608.* If the search is authorized (606-Yes), the donor characteristics are transmitted to the LIMS application (Fig. 1) so that stem cell database 52 can be searched for matching donor records.

*Steps 610.* In step 610, a search for stem cells having characteristics that match those of the patient is performed by data query routine 48 of stem cell tracking module 44 (Fig. 1). To facilitate efficient searching, stem cell database 52 is preferably a relational database. However, such a technical requirement is not necessary to facilitate the methods of the present invention and, in fact, stem cell database 52 could be a flat file with no database schema or hierarchical organization.

*Steps 612 and 614.* If a match is found (612-Yes), process control passes to step 616 where a determination is made as to whether the match is public or private. Otherwise (612-No), the failure is communicated to CRM application 244 (614).

*Steps 616 and 618.* Step 616 is reached if there is a match. When a match arises, a determination is made as to whether the match is public or private. If the match is private (616-No), the match is considered unavailable to a patient other than the donor of the matching stem cell unit or a close kin of the matching stem cell unit. Thus, in such instances, the search fails and such failure is communicated to CRM application 244 (618).

*Step 620.* If the matching stem cell unit is public (620-Yes), all or a portion of the matching stem cell unit is allocated to the patient. The methods described in this section can be combined with embodiments of the invention described in Sections 5.2 and 5.3. For instance, the matching stem cell unit could comprise multiple stem cell transplant units. Thus, in some embodiments of step 620, a portion of the matching stem cell unit could be allocated to the patient. When this is the case, the number of transplant units available field 64 in the matching donor record 54 is deducted by the number of stem cell transplant units allocated to the patient.

### 5.6. DISEASES THAT CAN BE TREATED USING STEM CELL TRANSPLANTS

Diseases treatable by stem cell transplantation include, but are not limited to, stem cell disorders (*e.g.,* aplastic anemia, Fanconi Anemia, paroxysmal nocturnal hemoglobinuria), acute leukemias (*e.g.,* acute lymphoblastic leukemia, acute myelogenous leukemia, acute biphenotypic leukemia, acute undifferentiated leukemia), chronic leukemias (*e.g.,* chronic myelogenous leukemia, chronic lymphocytic leukemia, juvenile chronic myelogenous leukemia, juvenile myelomonocytic leukemia), myeloproliferative disorders (*e.g.,* acute myelofibrosis, agnogenic myeloid metaplasia, polycythemia vera, essential thrombocythemia), myelodysplastic syndromes (*e.g.,* refractory anemia, refractory anemia with ringed sideroblasts, refractory anemia with excess blasts, refractory anemia with excess blasts in transformation, chronic myelomonocytic Leukemia), lymphoproliferative disorders (*e.g.,* non-Hodgkin's lymphoma, Hodgkin's disease, prolymphocytic Leukemia) inherited erythrocyte abnormalities (*e.g.,* beta thalassemia major, pure red cell aplasia, sickle cell disease), liposomal storage diseases (*e.g.,* mucopolysaccharidoses, Hurler syndrome, Scheie syndrome, Hunter's syndrome, Sanfilippo syndrome, Morquio syndrome, Maroteaux-Lamy syndrome, Sly syndrome, beta-glucuronidase deficiency, adrenoleukodystrophy, mucolipidosis II, Krabbe disease, Gaucher's disease, Niemann-Pick disease, Wolman disease, metachromatic leukodystrophy), histiocytic disorders (*e.g.,* familial erythrophagocytic lymphohistiocytosis, histiocytosis-X, hemophagocytosis), phagocyte disorders (Chediak-Higashi syndrome, chronic granulomatous disease, neutrophil actin deficiency, reticular dysgenesis) congenital immune system disorders (*e.g.,* ataxia-telangiectasia, kostmann syndrome, leukocyte adhesion deficiency, DiGeorge syndrome, bare lymphocyte syndrome, Omenn's syndrome, severe combined immunodeficiency (SCID), SCID with adenosine deaminase deficiency, absence of T and B Cell SCID, absence of T Cell, normal B Cell SCID, common Variable Immunodeficiency, Wiskott-Aldrich syndrome, x-linked lymphoproliferative disorder, inherited platelet abnormalities (*e.g.,* amegakaryocytosis / congenital thrombocytopenia), plasma cell disorders, (*e.g.,* multiple myeloma, plasma cell leukemia, Waldenstrom's macroglobulinemia), other inherited disorders (*e.g.,* Lesch-Nyhan syndrome, cartilage-hair hypoplasia, Glanzmann Thrombasthenia, Osteopetrosis), and other malignancies (*e.g.,* breast cancer, Ewing sarcoma, neuroblastoma, and renal cell carcinoma).

Stem cells are also expected to have an anti-inflammatory effect when administered to an individual experiencing inflammation. In a preferred embodiment, stem cells may be used to treat any disease, condition or disorder resulting from, or associated with, inflammation. The inflammation may be present in any organ or tissue, for example, muscle; nervous system, comprising the brain, spinal cord and peripheral nervous system; vascular tissues, comprising cardiac tissue; pancreas; intestine or other organs of the digestive tract; lung; kidney; liver; reproductive organs; endothelial tissue, or endodermal tissue.

Stem cells may also be used to treat immune-related disorders, particularly autoimmune disorders, comprising those associated with inflammation. Thus, in certain embodiments, the invention provides a method of treating an individual having an autoimmune disease or condition, comprising administering to such individual a therapeutically effective amount of stem cells, wherein said disease or disorder can be, but is not limited to, diabetes, amylotrophic lateral sclerosis, myasthenia gravis, diabetic neuropathy or lupus. Cord blood or cord blood-derived stem cells may also be used to treat acute or chronic allergies, *e.g.,* seasonal allergies, food allergies, allergies to self-antigens, *etc.*

In certain embodiments, the disease or disorder includes, but is not limited to, any of the diseases or disorders disclosed herein, comprising, but not limited to aplastic anemia, myelodysplasia, myocardial infarction, seizure disorder, multiple sclerosis, stroke, hypotension, cardiac arrest, ischemia, inflammation, age-related loss of cognitive function, radiation damage, cerebral palsy, neurodegenerative disease, Alzheimer's disease, Parkinson's disease, Leigh disease, AIDS dementia, memory loss, amyotrophic lateral sclerosis (ALS), ischemic renal disease, brain or spinal cord trauma, heart-lung bypass, glaucoma, retinal ischemia, retinal trauma, lysosomal storage diseases, such as Tay-Sachs, Niemann-Pick, Fabry's, Gaucher's, Hunter's, and Hurler's syndromes, as well as other gangliosidoses, mucopolysaccharidoses, glycogenoses, inborn errors of metabolism, adrenoleukodystrophy, cystic fibrosis, glycogen storage disease, hypothyroidism, sickle cell anemia, Pearson syndrome, Pompe's disease, phenylketonuria (PKU), porphyrias, maple syrup urine disease, homocystinuria, mucoplysaccharidosis, chronic granulomatous disease and tyrosinemia, Tay-Sachs disease, cancer, tumors or other pathological or neoplastic conditions.

In other embodiments, the stem cells may be used in the treatment of any kind of injury due to trauma, particularly trauma involving inflammation. Examples of such trauma-related conditions include central nervous system (CNS) injuries, comprising injuries to the brain, spinal cord, or tissue surrounding the CNS injuries to the peripheral nervous system (PNS); or injuries to any other part of the body. Such trauma may be caused by accident, or may be a normal or abnormal outcome of a medical procedure such as surgery or angioplasty. Trauma may also be the result of the rupture, failure or occlusion of a blood vessel, such as in a stroke or phlebitis. In specific embodiments, the stem cells may be used in autologous or heterologous tissue regeneration or replacement therapies or protocols, comprising, but not limited to treatment of corneal epithelial defects, cartilage repair, facial dermabrasion, mucosal membranes, tympanic membranes, intestinal linings, neurological structures (*e.g.,* retina, auditory neurons in basilar membrane, olfactory neurons in olfactory epithelium), burn and wound repair for traumatic injuries of the skin, or for reconstruction of other damaged or diseased organs or tissues.

In a specific embodiment, the disease or disorder is aplastic anemia, myelodysplasia, leukemia, a bone marrow disorder or a hematopoietic disease or disorder. In another specific embodiment, the subject is a human.

In another embodiment, the invention provides a method of treating an individual having a disease, disorder or condition associated with or resulting from inflammation. In a specific embodiment, the disease, disorder or condition is a neurological disease, disorder or condition. In a more specific embodiment, the neurological disease is amylotrophic lateral sclerosis (ALS). In another more specific embodiment, the neurological disease is Parkinson's disease. In another specific embodiment, the disease is a vascular or cardiovascular disease. In a more specific embodiment, the disease is atherosclerosis. In another specific embodiment, the disease is diabetes.

### 5.7. METHODS OF ISOLATING AND CULTURING PLACENTA IN ORDER TO OBTAIN STEM CELLS

In some embodiments, stem cells are recovered from a mammalian placenta using any of the techniques disclosed in United States Patent Application 10/366,671 entitled "Embryonic stem cells derived from post-partum mammalian placenta, and uses and methods of treatment using said cells", United States Patent Application 10/411,655 entitled "Modulation of stem and progenitor cell differentiation, assays, and uses thereof," PCT publication WO 02/064755 A2, published August 22, 2002; or PCT publication WO 02/46373 A1, published June 13, 2002, each of which is incorporated herein by reference in its entirety. Illustrative techniques for how stem cells are obtained from placenta are disclosed in the following subsections. Further, Example 1, found in Section 6, provides an example of how stem cells are obtained from placental tissue in accordance with one embodiment of the present invention.

### 5.7.1 PRETREATMENT OF PLACENTA

A mammalian placenta is recovered shortly after its expulsion after birth and, in certain embodiments, the cord blood in the placenta is recovered. In certain embodiments, the placenta is subjected to a conventional cord blood recovery process. Such cord blood recovery may be obtained commercially, *e.g.,* LifeBank Inc., Cedar Knolls, N.J., ViaCord, Cord Blood Registry and Cryocell. The cord blood can be drained shortly after expulsion of the placenta. In other embodiments, the placenta is pretreated according to the methods disclosed in co-pending application No. 10/076,180, filed February 13, 2002, which is incorporated herein by reference in its entirety.

### 5.7.2 EXSANGUINATION OF PLACENTA AND REMOVAL OF RESIDUAL CELLS

As disclosed in PCT publication WO 02/064755, published August 22, 2002, which is incorporated herein by reference in its entirety, the placenta after birth contains quiescent cells that can be activated if the placenta is properly processed after birth. For example, after expulsion from the womb, the placenta is exsanguinated as quickly as possible to prevent or minimize apoptosis. Subsequently, as soon as possible after exsanguination the placenta is perfused to remove blood, residual cells, proteins, factors and any other materials present in the organ. Material debris can also be removed from the placenta. Perfusion is normally continued with an appropriate perfusate for at least two to more than twenty-four hours. The placenta can therefore readily be used as a rich and abundant source of embryonic-like stem cells. The human placental stem cells produced by the exsanguinated, perfused and/or cultured placenta are pluripotent stem cells that can readily be differentiated into desired cell types.

Stem or progenitor cells, comprising, but not limited to, embryonic-like stem cells, can be recovered from a placenta that is exsanguinated, *e.g.,* completely drained of the cord blood remaining after birth and/or a conventional cord blood recovery procedure. The methods for exsanguination of the placenta and removal of residual cells can be accomplished using any method known in the art, *e.g.,* the methods disclosed in PCT publication WO 02/064755, published August 22, 2002, which is incorporated herein by reference in its entirety.

### 5.7.3. CULTURING PLACENTA

After exsanguination and a sufficient time of perfusion of the placenta, the embryonic-like stem cells are observed to migrate into the exsanguinated and perfused microcirculation of the placenta where they are collected, preferably by washing into a collecting vessel by perfusion. In some embodiments, the placenta is cultured, and the cells propagated are monitored, sorted and/or characterized according to the methods described in PCT publication WO 02/064755, published August 22, 2002, which is incorporated herein by reference in its entirety. In still other embodiments, the placenta is not cultured. Rather, stem cells are collected by washing into a collection vessel by perfusion.

### 5.7.4 COLLECTION OF CELLS FROM THE PLACENTA

After exsanguination and perfusion of the placenta, embryonic-like stem cells migrate into the drained, empty microcirculation of the placenta where they are collected, preferably by collecting the effluent perfusate in a collecting vessel. In some instances, cells cultured in the placenta are isolated from the effluent perfusate using techniques known by those skilled in the art, such as, for example, centrifugation (*e.g.,* conventional centrifugation and/or density gradient centrifugation), magnet cell separation, flow cytometry, or other well known cell separation or sorting methods, and sorted.

In a specific embodiment, the embryonic-like stem cells are collected from the placenta and, in certain embodiments, preserved, according to the methods described in PCT publication WO 02/064755, published August 22, 2002, which is incorporated herein by reference in its entirety.

### 5.8. STEM CELL DATABASE AND CRM DATABASE ARCHITECTURE

In some embodiments, stem cell database 52 and/or CRM database 250 is a data warehouse. Data warehouses are typically structured as either relational databases or multidimensional data cubes. In this section, exemplary databases 52 and/or database 250 having a relational database or a multidimensional data cube architecture are described. For more information on relational databases and multidimensional data cubes, see Berson and Smith, 1997, Data Warehousing, Data Mining and OLAP, McGraw-Hill, New York; Freeze, 2000, Unlocking OLAP with Microsoft SQL Server and Excel 2000, IDG Books Worldwide, Inc., Foster City, California; and Thomson, 1997, OLAP Solutions: Building Multidimensional Information Systems, Wiley Computer Publishing, New York. In addition, it will be appreciated that in some embodiments database 52 and/or database 250 does not have a formal hierarchical structure.

### 5.8.1 DATA ORGANIZATION

Databases have typically been used for operational purposes (OLTP), such as order entry, accounting and inventory control. More recently, corporations and scientific projects have been building databases, called data warehouses or large on-line analytical processing (OLAP) databases, explicitly for the purposes of exploration and analysis. The "data warehouse" can be described as a subject-oriented, integrated, time-variant, nonvolatile collection of data in support of management decisions. Data warehouses are built using both relational databases and specialized multidimensional structures called data cubes. In some embodiments database 52 and/or database 250 is a datacube or a relational database.

### 5.8.2 RELATIONAL DATABASES

Relational databases organize data into tables where each row corresponds to a basic entity or fact and each column represents a property of that entity. For example, a table may represent transactions in a bank, where each row corresponds to a single transaction, and each transaction has multiple attributes, such as the transaction amount, the account balance, the bank branch, and the customer. The relational table is referred to as a relation, a row as a tuple, and a column as an attribute or field. The attributes within a relation can be partitioned into two types: dimensions and measures. Dimensions and measures are similar to independent and dependent variables in traditional analysis. For example, the bank branch and the customer would be dimensions, while the account balance would be a measure. A single relational database will often describe many heterogeneous but interrelated entities. For example, a database designed for a coffee chain might maintain information about employees, products, and sales. The database schema defines the relations in a database, the relationships between those relations, and how the relations model the entities of interest.

### 5.8.3 DATA CUBE

A data warehouse can be constructed as a relational database using either a star or snowflake schema and will provide a conceptual model of a multidimensional data set. Each axis in the corresponding data cube represents a dimension in a relational schema and consists of every possible value for that dimension. For example, an axis corresponding to states would have fifty values, one for each state. Each cell in the data cube corresponds to a unique combination of values for the dimensions. For example, if there are two dimensions, "State" and "Product", then there would be a cell for every unique combination of the two, *e.g.,* one cell each for (California, Tea), (California, Coffee), (Florida, Tea), (Florida, Coffee), *etc.* Each cell contains one value per measure of the data cube. So if product production and consumption information is needed, then each cell would contain two values, one for the number of products of each type consumed in that state, and one for the number of products of each type produced in that state. Dimensions within a data warehouse are often augmented with a hierarchical structure. If each dimension has a hierarchical structure, then the data warehouse is not a single data cube but rather a lattice of data cubes.

### 6. EXAMPLES

### 6.1. EXAMPLE 1: ANALYSIS OF CELLS OBTAINED BY PERFUSION AND INCUBATION OF PLACENTA

The following example describes an analysis of cells that can be obtained by perfusion and incubation of placenta according to the methods of the invention.

### 6.1.1 MATERIALS AND METHODS

Placenta donors can be recruited from expectant mothers that enrolled in umbilical cord blood banking programs and provided informed consent permitting the use of the exsanguinated placenta following recovery of cord blood for research purposes. Donor data may be confidential. These donors can also be permitted to use of blinded data generated from the normal processing of their umbilical cord blood specimens for cryopreservation. This allows comparison between the composition of the collected cord blood and the effluent perfusate recovered using the experimental method described below.

Following exsanguination of cord blood from the umbilical cord and placenta, the placenta is stored at room temperature and delivered to the laboratory within four to twenty-four hours. Placenta can be placed in sterile, insulated containers at room temperature and delivered to the laboratory within 4 hours of birth. Placentas are discarded if, on inspection, they had evidence of physical damage such as fragmentation of the organ or avulsion of umbilical vessels. Placentas are maintained at room temperature (23±2°C) or refrigerated (4°C) in sterile containers for 2 to 20 hours. Periodically, the placentas can be immersed and washed in sterile saline at 25+3°C to remove any visible surface blood or debris.

The umbilical cord can be transected approximately 5 cm from its insertion into the placenta and the umbilical vessels are cannulated with TEFLON® or polypropylene catheters connected to a sterile fluid path allowing bi-directional perfusion of the placenta and recovery of the effluent fluid. The methods described hereinabove enable all aspects of placental conditioning, perfusion and effluent collection to be performed under controlled ambient atmospheric conditions as well as real-time monitoring of intravascular pressure and flow rates, core and perfusate temperatures and recovered effluent volumes. A range of conditioning protocols can be evaluated over a 24-hour postpartum period, and the cellular composition of the effluent fluid can be analyzed by flow cytometry, light microscopy and colony forming unit assays.

### 6.1.2 PLACENTAL CONDITIONING

The donor placentas are processed at room temperature within 12 to 48 hours after delivery. Before processing, the membranes are removed and the maternal site washed clean of residual blood. The umbilical vessels are cannulated with catheters made from 20 gauge Butterfly needles use for blood unit collection.

The donor placentas are maintained under varying conditions such as maintenance at 5-37° 5% CO₂, pH 7.2 to 7.5, preferably pH 7.45, in an attempt to simulate and sustain a physiologically compatible environment for the proliferation and recruitment of residual embryonic-like stem cells. The cannula are flushed with IMDM serum-free medium (GibcoBRL, NY) containing 2U/ml heparin (Elkins-Sinn, NJ). Perfusion of the placenta continues at a rate of 50 ml per minute until approximately 150 ml of perfusate is collected. This volume of perfusate is optionally labeled "early fraction." Continued perfusion of the placenta at the same rate results in the collection of a second fraction of approximately 150 ml and is optionally labeled "late fraction." During the course of the procedure, the placenta is gently massaged to aid in the perfusion process and to assist in the recovery of cellular material. Effluent fluid is collected from the perfusion circuit by both gravity drainage and aspiration through the arterial cannula.

Placentas are then perfused with heparinized (2U/ml) Dulbecco's modified Eagle Medium (H.DMEM) at the rate of 15 ml/minute for 10 minutes and the perfusates is collected from the maternal sites within one hour and the nucleated cells counted. The perfusion and collection procedures are repeated once or twice until the number of recovered nucleated cells falls below 100/ml. The perfusates are pooled and subjected to light centrifugation to remove platelets, debris and de-nucleated cell membranes. The nucleated cells are then isolated by Ficoll-Hypaque density gradient centrifugation and after washing, resuspended in H.DMEM. For isolation of the adherent cells, aliquots of 5-10 x 10⁶ cells are placed in each of several T-75 flasks and cultured with commercially available Mesenchymal Stem Cell Growth Medium (MSCGM) obtained from BioWhittaker, and placed in a tissue culture incubator (37°C, 5% CO₂). After 10 to 15 days, the non-adherent cells are removed by washing with PBS, which is then replaced by MSCGM. The flasks are examined daily for the presence of various adherent cell types and, in particular, for identification and expansion of clusters of fibroblastoid cells.

### 6.1.3 CELL RECOVERY AND ISOLATION

Cells are recovered from the perfusates by centrifugation at 5000 x g for 15 minutes at room temperature. This procedure serves to separate cells from contaminating debris and platelets. The cell pellets are resuspended in IMDM serum-free medium containing 2U/ml heparin and 2mM EDTA (GibcoBRL, NY). The total mononuclear cell fraction is isolated using Lymphoprep (Nycomed Pharma, Oslo, Norway) according to the manufacturer's recommended procedure and the mononuclear cell fraction is resuspended. Cells are counted using a hemocytometer. Viability is evaluated by trypan blue exclusion. Isolation of mesenchymal cells is achieved by "differential trypsinization," using a solution of 0.05% trypsin with 0.2% EDTA (Sigma, St. Louis MO). Differential trypsinization is possible because fibroblastoid cells detached from plastic surfaces within about five minutes whereas the other adherent populations require incubation for more than 20-30 minutes. The detached fibroblastoid cells are harvested following trypsinization and trypsin neutralization, using Trypsin Neutralizing Solution (TNS, BioWhittaker). The cells are washed in H.DMEM and resuspended in MSCGM.

Flow cytometry is carried out using a Becton-Dickinson FACSCalibur instrument. FITC and PE labeled monoclonal antibodies (mAbs), selected on the basis of known markers for bone marrow-derived MSC (mesenchymal stem cells), can be purchased from B.D. and Caltag laboratories (South San Francisco, CA.). Reactivities of the mAbs in their cultured supernatants are detected by FITC or PE labeled F(ab)'2 goat anti-mouse antibodies. Lineage differentiation is carried out using commercially available induction and maintenance culture media (BioWhittaker), used as per manufacturer's instructions.

### 6.1.4 ISOLATION OF PLACENTAL EMBRYONIC-LIKE STEM CELLS

Microscopic examination of the adherent cells in the culture flasks should reveal morphologically different cell types. Spindle-shaped cells, round cells with large nuclei and numerous perinuclear small vacuoles, and star-shaped cells with several projections (through one of which star-shaped cells were attached to the flask) can be observed adhering to the culture flasks. Although no attempts are made to further characterize such adherent cells, similar cells can be observed in the culture of bone marrow, cord and peripheral blood, and therefore are considered to be non-stem cell-like in nature. The fibroblastoid cells, appearing last as clusters, are candidates for being MSC (mesenchymal stem cells) and are isolated by differential trypsinization and subcultured in secondary flasks. Phase microscopy of the rounded cells, after trypsinization, may reveal that the cells are highly granulated; indistinguishable from the bone marrow-derived MSC produced in the laboratory or purchased from BioWhittaker. When subcultured, the placenta-derived embryonic-like stem cells, in contrast to their earlier phase, may adhere within hours, assume characteristic fibroblastoid shape, and form a growth pattern identical to the reference bone marrow-derived MSC. During subculturing and refeeding, moreover, the loosely bound mononuclear cells can be washed out and the cultures remaining may be homogeneous and devoid of any visible non-fibroblastoid cell contaminants.

### 6.1.5 RESULTS

The expression of CD34-, CD38-, and other stem cell-associated surface markers on early and late fraction purified mononuclear cells can be assessed by flow cytometry. Recovered, sorted cells can be washed in PBS and then double-stained with antiCD34 phycoerythrin and anti-CD38 fluorescein isothiocyanate (Becton Dickinson, Mountain View, CA).

Cell isolation can be achieved by using magnetic cell separation, such as for example, Auto Macs (Miltenyi). Preferably, CD34+ cell isolation is performed first.

### 6.2. EXAMPLE 2: EXEMPLARY SYSTEM

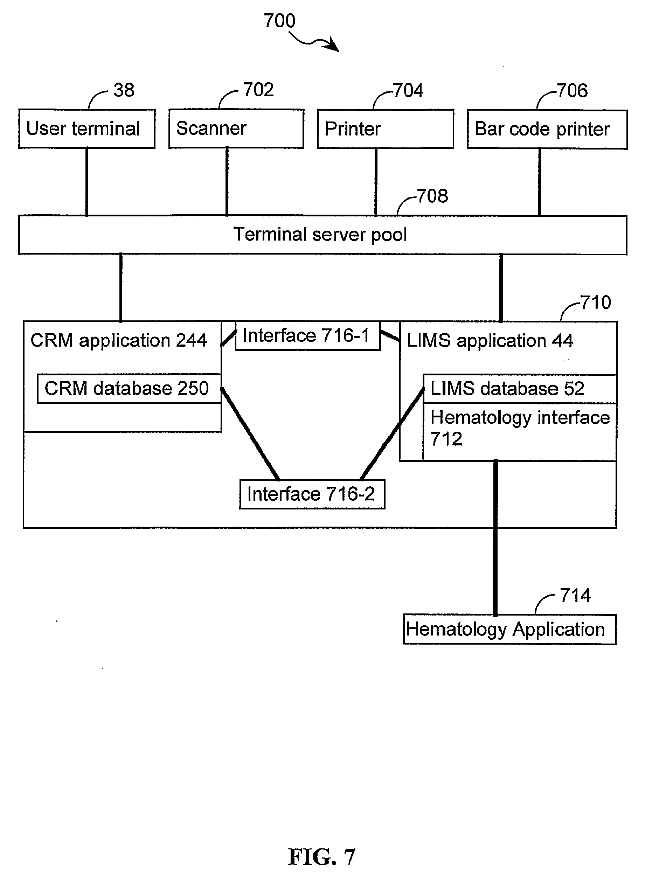

Referring to Fig. 7, there is shown an exemplary system 700 in accordance with one embodiment of the present invention. Exemplary system 700 includes a one or more user terminals 38, scanners 702, printers 704, and bar code printers 706 that are interfaced with a terminal server pool 708. Terminal server pool 708 hosts and/or is in electronic communication with CRM application 244 and LIMS application 44. In some embodiments, CRM application 244 and LIMS application 44 are hosted by different servers. In the embodiment illustrated in Fig. 7, donor anonymity is preserved between CRM application 244 and LIMS application 44 through interface 716. Interface allows for the exchange of certain information between CRM application 244 and LIMS application 44 and the exchange of certain information between CRM database 250 and LIMS database. In some embodiments, interface 716 passes the information listed in Table 1 below from LIMS application 44 to CRM application 244. Note that the information in Table 1 does not include sensitive information such as the results of an HIV assay.

**Table 1: Information passed from LIMS application 44 to CRM application 244 by interface 716 in an exemplary embodiment.**

| |
|---|
| Mother Contact ID |
| Baby Collection ID |
| Check-in Time |
| Patient Status |
| Private Indicator |
| Actual Birth Date / Time |
| Placenta Expulsion Date / Time |
| Mother Race |
| Father Race |
| |
| **PROCESS INDICATORS** |
| High Risk Indicator |
| Maternal Blood Process Indicator |
| Transferred |
| Cord Blood Process Indicator |
| Approved |
| Approved with Follow up |
| Not Approved |
| Experimental |
| Placenta Process Indicator |
| Approved |
| Approved with Follow up |
| Not Approved |
| Experimental |
| Tissue Process Indicator |
| Approved |
| Approved with Follow up |
| Not Approved |
| Experimental |
| Document Variances |
| In-Utero Flag |
| Donor Delivery Record Variances |
| Not Labeled |
| Labeled Incorrectly |
| Missing information |
| |
| **PRODUCT COLLECTION VARIANCES** |
| Maternal Blood Variances |
| Not all tubes received |
| Not Labeled |
| Labeled incorrectly |
| Not full |
| Not closed properly |
| Cord Blood Variances |
| Not Labeled |
| Labeled incorrectly |
| Not clamped |
| Collection bag not sealed properly |
| Satellite bags missing |
| Needle is not shielded properly |
| Placenta Variances |
| Not Labeled |
| Labeled incorrectly |
| Outer bag not sealed properly |

In some embodiments, interface 716 passes the information listed in Table 2 below from CRM application 244 to LIMS application.

**Table 2: Information passed from CRM application 244 to LIMS application 44 by interface 716 in an Exemplary embodiment.**

| |
|---|
| Cord Blood Final Total Nucleated Cells |
| Cord Blood Freezer Date |
| Placenta Final Total Nucleated Cells |
| Placenta Freezer Date |

In some embodiments, LIMS application 44 further includes a hematology interface 712 for interfacing with a hematology application 714. Hematology application 714 is used to characterize donated stem cell units (*e.g.,* determine HLA type). Then, the characterization of the donated stem cell unit is uploaded into LIMS database 52 by hematology interface 712.

### 7. CONCLUSION

The foregoing descriptions of specific embodiments of the present invention are presented for purposes of illustration and description. They are not intended to be exhaustive or to limit the invention to the precise forms disclosed, obviously many modifications and variations are possible in view of the above teachings.

The present invention can be implemented as a computer program product that comprises a computer program mechanism embedded in a computer readable storage medium. For instance, the computer program product could contain the program modules shown in Fig. 1 and/or Fig. 2. These program modules can be stored on a RAM, a ROM, a disk, an ASIC, a PROM, a magnetic disk storage product, or any other computer readable data or program storage product. The software modules in the computer program product can also be distributed electronically, via the Internet or otherwise, by transmission of a computer data signal (in which the software modules are embedded) on a carrier wave.

All references cited herein are incorporated herein by reference in their entirety and for all purposes to the same extent as if each individual publication, patent or patent application was specifically and individually indicated to be incorporated by reference in its entirety for all purposes.

The citation of any publication is for its disclosure prior to the filing date and should not be construed as an admission that the present invention is not entitled to antedate such publication by virtue of prior invention.

### Preferred items:

1. A method of maintaining a stem cell registry, said method comprising:
   enrolling a donor in said stem cell registry;
   characterizing a plurality of placental stem cell transplant units from said donor, wherein said placental stem cell transplant units are stored as or within one or more placental stem cell units; and
   recording information about said plurality of placental stem cell transplant units obtained by said characterizing in said stem cell registry.
2. The method of item 1 wherein each placental stem cell transplant unit in said plurality of placental stem cell transplant units comprises between 25 x 10⁷ nucleated cells and 50 x 10⁷ nucleated cells.
3. The method of item 1 wherein each placental stem cell transplant unit in said plurality of placental stem cell transplant units comprises between 50 x 10⁷ nucleated cells and 75 x 10⁷ nucleated cells.
4. The method of item 1 wherein each placental stem cell transplant unit in said plurality of stem cell transplant units comprises between 75 x 10⁷ nucleated cells and 200 x 10⁷ nucleated cells.
5. The method of item 1, wherein said enrolling comprises storing donor identification information about said donor in said stem cell registry.
6. The method of item 1 wherein the stem cell registry comprises information about a plurality of stem cell units, the method further comprising:
   obtaining type information of a patient; and
   searching through said plurality of stem cell units for a stem cell unit having type information that matches the type information of the patient, wherein
      when a match is found between a stem cell unit in said plurality of stem cell units and said patient, the method further comprises:
         (i) allocating a number of placental stem cell transplant units from the matching stem cell unit in said plurality of stem cell units to said patient; and
         (ii) decrementing a number of placental stem cell transplant units of the matching stem cell unit available in the stem cell registry by the number of placental stem cell transplant units allocated to the patient.
7. The method of item 6 wherein the number of placental stem cell transplant units from the matching stem cell unit allocated to said patient is a function of the number of nucleated stem cells in each allocated placental stem cell transplant unit.
8. The method of item 6 wherein the number of placental stem cell transplant units from the matching stem cell unit allocated to said patient is a function of the number of nucleated stem cells in each allocated placental stem cell transplant unit and the weight of the patient.
9. The method of item 8 wherein between 0.5 x 10⁷ and 200 x 10⁷ nucleated cells per kilogram of patient body weight are allocated to said patient.
10. The method of item 8 wherein between 1 x 10⁷ and 50 x 10⁷ nucleated cells per kilogram of patient body weight are allocated to said patient.
11. The method of item 6 wherein the number of placental stem cell transplant units from the matching stem cell unit allocated to said patient is a function of the number of nucleated stem cells in each allocated placental stem cell transplant unit, the age of the patient, and the weight of the patient.
12. The method of item 1 wherein said donor is mammalian and wherein a first placental stem cell transplant unit in said plurality of placental stem cell transplant units from said donor comprises placental stem cells obtained from a post-partum placenta of the donor that has been exsanguinated and perfused.
13. The method of item 1 wherein the donor is human.
14. The method of item 1 wherein said stem cell registry comprises information about at least 100 stem cell units.
15. The method of item 1 wherein said stem cell registry comprises information about at least 1000 stem cell units.
16. The method of item 1 wherein said characterizing comprises:
   performing a nucleated cell count on a placental stem cell transplant unit in said plurality of placental stem cell transplant units,
   performing a leukocyte count on a placental stem cell transplant unit in said plurality of placental stem cell transplant units,
   performing a mononuclear cell count on a placental stem cell transplant unit in said plurality of placental stem cell transplant units,
   performing a CD34-positive cell count on a placental stem cell transplant unit in said plurality of placental stem cell transplant units,
   performing a CD3-positive cell count on a placental stem cell transplant unit in said plurality of placental stem cell transplant units,
   performing a colony-forming cell assay on a placental stem cell transplant unit in said plurality of placental stem cell transplant units,
   performing an infectious disease assay on a placental stem cell transplant unit in said plurality of placental stem cell transplant units, or
   determining an HLA blood type of a placental stem cell transplant unit in said plurality of placental stem cell transplant units.
17. The method of item 1, wherein said characterizing comprises separately characterizing each placental stem cell transplant unit in said plurality of placental stem cell transplant units.
18. The method of item 1 wherein
   said enrolling said donor in said stem cell registry comprises receiving a fee from said donor, the method further comprising:
   receiving a request from said donor or a family member of said donor for a placental stem cell transplant unit in said plurality of placental stem cell transplant units; and
   allocating said placental stem cell transplant unit to said donor or said family member of said donor.
19. A computer program product for use in conjunction with a computer system, the computer program product comprising a computer readable storage medium and a computer program mechanism embedded therein, the computer program mechanism comprising:
   a stem cell database comprising information about stem cells; and
   a stem cell tracking module, the stem cell tracking module comprising a data entry routine that comprises:
      instructions for enrolling a donor in said stem cell database;
      instructions for receiving a characterization of a plurality of placental stem cell transplant units that originate from said donor; and
      instructions for recording said characterization in said stem cell database.
20. The computer program product of item 19 wherein a placental stem cell transplant unit in said plurality of placental stem cell transplant units comprises between 25 x 10⁷ nucleated cells and 50 x 10⁷ nucleated cells.
21. The computer program product of item 19 wherein a placental stem cell transplant unit in said plurality of placental stem cell transplant units comprises between 50 x 10⁷ nucleated cells and 75 x 10⁷ nucleated cells.
22. The computer program product of item 19 wherein a placental stem cell transplant unit in said at plurality of placental stem cell transplant units comprises between 75 x 10⁷ nucleated cells and 200 x 10⁷ nucleated cells.
23. The computer program product of item 19, wherein said instructions for enrolling comprise instructions for storing donor identification information about said donor in said stem cell database.
24. The computer program product of item 19, wherein the stem cell tracking module further includes a data query routine that comprises:
   instructions for obtaining type information of a patient; and
   instructions for searching through information about a plurality of stem cell units in said stem cell database for a stem cell unit having type information that matches the type information of the patient, wherein
      when a match is found between a stem cell unit in said plurality of stem cell units and said patient, the data query routine further comprises:
         instructions for allocating a number of placental stem cell transplant units in the matching stem cell unit to said patient; and
         instructions for decrementing a number of placental stem cell transplant units of the matching stem cell unit available in the stem cell registry by the number of placental stem cell transplant units allocated to said patient.
25. The computer program product of item 24 wherein the number of placental stem cell transplant units from the matching stem cell unit allocated to said patient is a function of the number of nucleated stem cells in each allocated placental stem cell transplant unit.
26. The computer program product of item 24 wherein the number of placental stem cell transplant units from the matching stem cell unit allocated to said patient is a function of the number of nucleated stem cells in each allocated placental stem cell transplant unit and the weight of the patient.
27. The computer program product of item 26 wherein between 0.5 x 10⁷ and 200 x 10⁷ nucleated cells per kilogram of patient body weight are allocated to said patient.
28. The computer program product of item 26 wherein between 1 x 10⁷ and 50 x 10⁷ nucleated cells per kilogram of patient body weight are allocated to said patient.
29. The computer program product of item 24 wherein the number of placental stem cell transplant units from the matching stem cell unit allocated to said patient is a function of the number of nucleated stem cells in each allocated placental stem cell transplant unit, the age of the patient, and the weight of the patient.
30. The computer program product of item 19 wherein said donor is mammalian and said stem cell unit from said donor comprises stem cells obtained from a post-partum placenta of the donor that has been exsanguinated and perfused.
31. The computer program product of item 28 wherein the donor is human.
32. The computer program product of item 19 wherein said stem cell database comprises information about at least 100 stem cell units.
33. The computer program product of item 19 wherein said stem cell database comprises information about at least 1000 stem cell units.
34. The computer program product of item 19 wherein said instructions for receiving comprise:
   instructions for receiving a nucleated cell count on a placental stem cell transplant unit in said plurality of placental stem cell transplant units,
   instructions for receiving a leukocyte count on a placental stem cell transplant unit in said plurality of placental stem cell transplant units,
   instructions for receiving a mononuclear cell count on a placental stem cell transplant unit in said plurality of placental stem cell transplant units,
   instructions for receiving a CD34-positive cell count on a placental stem cell transplant unit in said plurality of placental stem cell transplant units,
   instructions for receiving a CD3-positive cell count on a placental stem cell transplant unit in said plurality of placental stem cell transplant units,
   instructions for receiving a colony-forming cell assay on a placental stem cell transplant unit in said plurality of placental stem cell transplant units,
   instructions for receiving the results of an infectious disease assay on a placental stem cell transplant unit in said plurality of placental stem cell transplant units, or
   instructions for receiving the results of an HLA blood type assay of a placental stem cell transplant unit in said plurality of placental stem cell transplant units.
35. The computer program product of item 19, the data entry routine further comprising:
   instructions for receiving a characterization of each placental stem cell transplant unit in said plurality of placental stem cell transplant units.
36. The computer program product of item 19, wherein
   said instructions for enrolling said donor in said stem cell database comprise instructions for receiving a fee from said donor, and wherein the stem cell tracking module further includes a data query routine that comprises:
      instructions for receiving a request from said donor or a family member for a placental stem cell transplant unit in said plurality of placental stem cell transplant units; and
      instructions for allocating a placental stem cell transplant unit in said plurality of placental stem cell transplant units to said donor or said family member of said donor.
37. A computer system comprising:
   a central processing unit;
   a memory, coupled to the central processing unit, the memory storing:
      a stem cell database comprising information about stem cell units; and
      a stem cell tracking module, the stem cell tracking module comprising a data entry routine that comprises:
         instructions for enrolling a donor in said stem cell database;
         instructions for receiving a characterization of a plurality of placental stem cell transplant units that originate from said donor; and
         instructions for recording information about said plurality of placental stem cell transplant units in said stem cell database.
38. The computer system of item 37 wherein each placental stem cell transplant unit in said plurality of placental stem cell transplant units comprises between 25 x 10⁷ nucleated cells and 50 x 10⁷ nucleated cells.
39. The computer system of item 37 wherein each placental stem cell transplant unit in said plurality of placental stem cell transplant units comprises between 50 x 10⁷ nucleated cells and 75 x 10⁷ nucleated cells.
40. The computer system of item 37 wherein each placental stem cell transplant unit in said of plurality of placental stem cell transplant units comprises between 75 x 10⁷ nucleated cells and 200 x 10⁷ nucleated cells.
41. The computer system of item 37, wherein said instructions for enrolling comprise instructions for storing donor identification information about said donor in said stem cell database.
42. The computer system of item 37, wherein the stem cell tracking module further includes a data query routine that comprises:
   instructions for obtaining type information of a patient; and
   instructions for searching through information about a plurality of stem cell units that is stored in said stem cell database for a stem cell unit having type information that matches the type information of the patient, wherein
      when a match is found between a stem cell unit in said plurality of stem cell units and said patient, the data query routine further comprises:
         instructions for allocating a number of placental stem cell transplant units from the matching stem cell unit in said plurality of stem cell units to said patient; and
         instructions for decrementing a number of placental stem cell transplant units of the matching stem cell unit available in the stem cell registry by the number of placental stem cell transplant units allocated to the patient.
43. The computer system of item 42 wherein the number of placental stem cell transplant units from the matching stem cell unit allocated to said patient is a function of the number of nucleated stem cells in each allocated placental stem cell transplant unit.
44. The computer system of item 43 wherein the number of placental stem cell transplant units from the matching stem cell unit allocated to said patient is a function of the number of nucleated stem cells in each allocated placental stem cell transplant unit and the weight of the patient.
45. The computer system of item 44 wherein between 0.5 x 10⁷ and 200 x 10⁷ nucleated cells per kilogram of patient body weight are allocated to said patient.
46. The computer system of item 44 wherein between 1 x 10⁷ and 50 x 10⁷ nucleated cells per kilogram of patient body weight are allocated to said patient.
47. The computer system of item 44 wherein the number of placental stem cell transplant units from the matching stem cell unit allocated to said patient is a function of the number of nucleated stem cells in each allocated stem cell transplant unit, the age of the patient, and the weight of the patient.
48. The computer system of item 37 wherein said donor is mammalian and said placental stem cell unit from said donor comprises stem cells obtained from a post-partum placenta of the donor that has been exsanguinated and perfused.
49. The computer system of item 37 wherein the donor is human.
50. The computer system of item 37 wherein said stem cell database comprises information about at least 100 stem cell units.
51. The computer system of item 37 wherein said stem cell database comprises information about at least 1000 stem cell units.
52. The computer system of item 37 wherein said instructions for receiving a characterization of said plurality of placental stem cell transplant units comprise:
   instructions for receiving a nucleated cell count on a placental stem cell transplant unit in said plurality of placental stem cell transplant units,
   instructions for receiving a leukocyte count on a placental stem cell transplant unit in said plurality of placental stem cell transplant units,
   instructions for receiving a mononuclear cell count on a placental stem cell transplant unit in said plurality of placental stem cell transplant units,
   instructions for receiving a CD34-positive cell count on a placental stem cell transplant unit in said plurality of placental stem cell transplant units,
   instructions for receiving a CD3-positive cell count on a placental stem cell transplant unit in said plurality of placental stem cell transplant units,
   instructions for receiving a colony-forming cell assay on a placental stem cell transplant unit in said plurality of placental stem cell transplant units, or
   instructions for receiving the results of an infectious disease assay on a placental stem cell transplant unit in said plurality of placental stem cell transplant units, or
   instructions for receiving the results of an HLA blood type assay of a placental stem cell transplant unit in said plurality of placental stem cell transplant units.
53. The computer system of item 37, the data entry routine further comprising:
   instructions for receiving a characterization of each placental stem cell transplant unit in said plurality of stem cell transplant units.
54. The computer system of item 37, wherein said instructions for receiving a characterization comprises instructions for receiving a separate characterization of each placental stem cell transplant unit in said plurality of placental stem cell transplant units.
55. The computer system of item 37, wherein
   said instructions for enrolling said donor in said stem cell database comprise instructions for receiving a fee from said donor, and wherein the stem cell tracking module further includes a data query routine that comprises:
   instructions for receiving a request from said donor or a family member for one or more placental stem cell transplant units in said plurality of placental stem cell transplant units; and
   instructions for allocating a placental stem cell transplant unit in said plurality of placental stem cell transplant units to said donor or said family member of said donor.
56. The computer system of item 55 wherein at least one placental stem cell transplant unit in said plurality of placental stem cell transplant units remains after the allocation of the one or more placental stem cell transplant units.
57. A method of maintaining a stem cell registry, said method comprising:
   enrolling a donor in said stem cell registry:
      characterizing a plurality of stem cell units from said donor, wherein a source of a first stem cell unit in said plurality of stem cell units differs from a source of a second stem cell unit in said plurality of stem cell units; and
      recording information about said plurality of stem cell units in said stem cell registry, wherein said information comprises an indication of at least one stem cell transplant unit in each stem cell unit in said plurality of stem cell units.
58. The method of item 57 wherein each said at least one stem cell transplant unit comprises between 25 x 10⁷ nucleated cells and 50 x 10⁷ nucleated cells.
59. The method of item 57 wherein each said at least one stem cell transplant unit comprises between 50 x 10⁷ nucleated cells and 75 x 10⁷ nucleated cells.
60. The method of item 57 wherein each said at least one stem cell transplant unit comprises between 75 x 10⁷ nucleated cells and 200 x 10⁷ nucleated cells.
61. The method of item 57, wherein said enrolling comprises storing donor identification information about said donor in said stem cell registry.
62. The method of item 57 wherein the stem cell registry comprises information about a plurality of stem cell units, each stem cell unit comprising a plurality of stem cell transplant units, the method further comprising:
   obtaining type information of a patient; and
   searching through said plurality of stem cell units for a stem cell unit having type information that matches the type information of the patient, wherein
      when a match is found between a stem cell unit in said plurality of stem cell units and said patient, the method further comprises:
         (i) allocating a number of stem cell transplant units from a stem cell unit in the matching stem cell unit in said plurality of stem cell units to said patient; and
         (ii) decrementing a number of stem cell transplant units in the stem cell unit of the matching stem cell unit available in the stem cell registry by the number of stem cell transplant units allocated to the patient.
63. The method of item 62 wherein the number of stem cell transplant units from the matching stem cell unit allocated to said patient is a function of the number of nucleated stem cells in each allocated stem cell transplant unit.
64. The method of item 63 wherein the number of stem cell transplant units from the matching stem cell unit allocated to said patient is a function of the number of nucleated stem cells in each allocated stem cell transplant unit and the weight of the patient.
65. The method of item 64 wherein between 0.5 x 10⁷ and 200 x 10⁷ nucleated cells per kilogram of patient body weight are allocated to said patient.
66. The method of item 64 wherein between 1 x 10⁷ and 50 x 10⁷ nucleated cells per kilogram of patient body weight are allocated to said patient.
67. The method of item 62 wherein the number of stem cell transplant units from the matching stem cell unit allocated to said patient is a function of the number of nucleated stem cells in each allocated stem cell transplant unit, the age of the patient, and the weight of the patient.
68. The method of item 57 wherein said donor is mammalian and wherein said first stem cell unit comprises placental stem cells obtained from a post-partum placenta of the donor that has been exsanguinated and perfused.
69. The method of item 57 wherein the donor is human.
70. The method of item 57 wherein said stem cell registry comprises information about at least 100 stem cell units.
71. The method of item 57 wherein said stem cell registry comprises information about at least 1000 stem cell units.
72. The method of item 57 wherein said characterizing comprises:
   performing a nucleated cell count on a stem cell unit in said plurality of stem cell units,
   performing a leukocyte count on a stem cell unit in said plurality of stem cell units,
   performing a mononuclear cell count on a stem cell unit in said plurality of stem cell units,
   performing a CD34-positive cell count on a stem cell unit in said plurality of stem cell units,
   performing a CD3-positive cell count on a stem cell unit in said plurality of stem cell units,
   performing a colony-forming cell assay on a stem cell unit in said plurality of stem cell units,
   performing an infectious disease assay on a stem cell unit in said plurality of stem cell units, or
   determining an HLA blood type of a stem cell unit in said plurality of placental stem cell units.
73. The method of item 57, wherein said characterizing comprises separately characterizing each stem cell unit in said plurality of stem cell units.
74. The method of item 57 wherein
   said enrolling said donor in said stem cell registry comprises receiving a fee from said donor, the method further comprising:
   receiving a request from said donor or a family member of said donor for a stem cell transplant unit in a stem cell unit in the plurality of stem cell units; and
   allocating said stem cell transplant unit to said donor or said family member of said donor.
75. The method of item 57 wherein said donor is mammalian and
   said source of said first stem cell unit is a post-partum placenta of the donor that has been exsanguinated and perfused, and
   said source of said second stem cell unit is the cord blood of said donor.
76. The method of item 57 wherein
   stem cells obtained from a post-partum placenta of the donor form one or more stem cell units in said plurality of stem cell units; and
   stem cells obtained from the cord blood of said donor form a different stem cell unit in said plurality of stem cell units.
77. A computer program product for use in conjunction with a computer system, the computer program product comprising a computer readable storage medium and a computer program mechanism embedded therein, the computer program mechanism comprising:
   a stem cell database comprising information about stem cells; and
   a stem cell tracking module, the stem cell tracking module comprising a data entry routine that comprises:
      instructions for enrolling a donor in said stem cell database;
      instructions for receiving a characterization of a plurality of stem cell units that originate from said donor, wherein a source of a first stem cell unit in the plurality of stem cell units differs from a source of a second stem cell unit in the plurality of stem cell units; and
      instructions for recording said characterization in said stem cell database, wherein said information includes an indication of at least one stem cell transplant unit in each stem cell unit in said plurality of stem cell units.
78. The computer program product of item 77 wherein a stem cell transplant unit in said plurality of stem cell units comprises between 25 x 10⁷ nucleated cells and 50 x 10⁷ nucleated cells.
79. The computer program product of items 77 wherein a stem cell transplant unit in said plurality of stem cell units comprises between 50 x 10⁷ nucleated cells and 75 x 10⁷ nucleated cells.
80. The computer program product of item 77 wherein a stem cell transplant unit in said plurality of stem cell units comprises between 75 x 10⁷ nucleated cells and 200 x 10⁷ nucleated cells.
81. The computer program product of item 77, wherein said instructions for enrolling comprise instructions for storing donor identification information about said donor in said stem cell database.
82. The computer program product of item 77, wherein the stem cell tracking module further includes a data query routine that comprises:
   instructions for obtaining type information of a patient; and
   instructions for searching through type information about a plurality of stem cell units in said stem cell database for a stem cell unit having type information that matches the type information of the patient, wherein
      when a match is found between a stem cell unit in said plurality of stem cell units and said patient, the data query routine further comprises:
         (i) instructions for allocating a number of stem cell transplant units in the matching stem cell unit to said patient; and
         (ii) instructions for decrementing a number of stem cell transplant units of the matching stem cell unit available in the stem cell registry by the number of stem cell transplant units allocated to said patient.
83. A computer system comprising:
   a central processing unit;
   a memory, coupled to the central processing unit, the memory storing:
      a stem cell database comprising information about stem cell units; and
      a stem cell tracking module, the stem cell tracking module comprising a data entry routine that comprises:
         instructions for enrolling a donor in said stem cell database;
         instructions for receiving a characterization of a plurality of stem cell units that originate from said donor, wherein a source of a first stem cell unit in said plurality of stem cell units differs from a source of a second stem cell unit in said plurality of stem cell units; and
         instructions for recording information about said plurality of stem cell units in said stem cell database, wherein said information includes an indication of at least one stem cell transplant unit in each stem cell unit in said plurality of stem cell units.
84. A computer program product for use in conjunction with a computer system, the computer program product comprising a computer readable storage medium and a computer program mechanism embedded therein, the computer program mechanism comprising:
   a customer relationship management database comprising information about a plurality of donors;
   a customer relationship management application that includes:
      (i) instructions for enrolling a donor in said customer relationship management database; and
      (ii) instructions for assigning a collection identifier number to said donor;
   a stem cell database comprising information about a plurality of stem cell units; and
   a stem cell tracking module that includes a data entry routine, the data entry routine comprising:
      (i) instructions for receiving the collection identifier number for said donor;
      (ii) instructions for receiving a characterization of a stem cell unit from said donor, wherein said characterization includes a characterization of stem cells from at least two different origins of said donor; and
      (iii) instructions for entering said characterization of said stem cell unit into said stem cell database.
85. The computer program product of item 84 wherein a first origin in said at least two different origins is cord blood of the donor and a second origin in said at least two different origins is the placenta of the donor.
86. The computer program product of item 84 wherein the placenta has been exsanguinated and perfused.
87. The computer program product of item 84 wherein said customer relationship management database includes a respective data entry for each donor in said plurality of donors, each said respective data entry comprising:
   a name of the donor;
   donor contact information;
   the collection identifier number associated with the donor;
   a cord blood cell count; and
   a placenta blood cell count.
88. The computer program product of item 84 wherein the stem cell database comprises a respective data entry for each donor in said plurality of donors, each said respective data entry comprising:
   the collection identifier number associated with the donor;
   a characterization of stem cells from a first origin of said donor; and
   a characterization of stem cells from a second origin of said donor.
89. The computer program product of item 88 wherein the first origin of said donor is cord blood and the second origin of said donor is the placenta associated with the donor.
90. The computer program product of item 89 wherein the stem cells from the second origin constitute a plurality of stem cell transplant units.
91. The computer program product of item 90 wherein each stem cell transplant unit in said plurality of stem cell transplant units comprises between 25 x 10⁷ nucleated cells and 50 x 10⁷ nucleated cells.
92. The computer program product of item 90 wherein each stem cell transplant unit in said plurality of stem cell transplant units comprises between 50 x 10⁷ nucleated cells and 75 x 10⁷ nucleated cells.
93. The computer program product of item 90 wherein each stem cell transplant unit in said plurality of stem cell transplant units comprises between 75 x 10⁷ nucleated cells and 200 x 10⁷ nucleated cells.
94. The computer program product of item 84 wherein said customer relationship management database comprises a flat file, a relational database, an on-line analytical processing database, or a hierarchical on-line analytical processing data cube.
95. The computer program product of item 84 wherein said stem cell database comprises a flat file, a relational database, an on line analytical processing database, or a hierarchical on-line analytical processing data cube.
96. The computer program product of item 84 wherein said customer relationship management database does not have an explicitly defined hierarchy.
97. The computer program product of item 84 wherein said stem cell database does not have an explicitly defined hierarchy.
98. The computer program product of item 84 wherein said customer relationship management database includes a relational star schema.
99. The computer program product of item 84 wherein said stem cell database includes a relational star schema.
100. The computer program product of item 84 wherein said stem cell tracking module further includes:
   instructions for receiving type information of a patient; and
   instructions for searching through said plurality of stem cell units for a stem cell unit having type information that matches the type information of the patient.
101. The computer program product of item 100 wherein said stem cell tracking module further includes instructions for receiving a search privilege, and wherein said instructions for searching are not performed when said search privilege is not received.
102. The computer program product of item 100 wherein, when a match is found between a stem cell unit in said plurality of stem cell units and said patient, said stem cell tracking module further includes instructions for granting the matching stem cell units to said patient when said matching stem cell units are public.
103. The computer program product of item 100 wherein the type information is human leukocyte antigen type.
104. A computer system comprising:
   a central processing unit;
   a network interface card for communicating with a remote computer; and
   a memory, coupled to the central processing unit, the memory storing:
      a customer relationship management database comprising information about a plurality of donors; and
      a customer relationship management application that includes:
         (i) instructions for enrolling a donor in said customer relationship management database;
         (ii) instructions for uniquely associating a collection identifier number to said donor; and
         (iii) instructions for transmitting said collection identifier number to a stem cell tracking module that is hosted by said remote computer.
105. A computer system comprising:
   a central processing unit;
   a network interface card for communicating with a remote computer; and
   a memory, coupled to the central processing unit, the memory storing:
      a stem cell database comprising information about a plurality of stem cell units; and
      a stem cell tracking module, the stem cell tracking module comprising a data entry routine that comprises:
         (i) instructions for receiving a collection identifier number from said remote computer, wherein the collection identifier number is uniquely associated with a donor;
         (ii) instructions for receiving a characterization of a stem cell unit from said donor, wherein said characterization includes a characterization of stem cells from at least two different origins of said donor; and
         (iii) instructions for updating the stem cell tracking module with the characterization of a stem cell unit from said donor
106. The computer system of item 105 wherein a first origin in said at least two different origins is cord blood of the donor and a second origin in said at least two different origins is the placenta of the donor.
107. The computer system of item 104 wherein said customer relationship management database includes a respective data entry for each donor in said plurality of donors, each said respective data entry comprising:
   a name of the donor;
   donor contact information;
   the collection identifier number associated with the donor;
   a cord blood cell count; and
   a placenta blood cell count.
108. The computer system of item 105 wherein the stem cell database comprises a respective data entry for each stem cell unit in said plurality of stem cell units, each said respective data entry comprising:
   a collection identifier number associated with a donor of the stem cell unit;
   a characterization of stem cells from a first origin of said donor; and
   a characterization of stem cells from a second origin of said donor.
109. The computer system of item 108 wherein the first origin of said donor is cord blood and the second origin of said donor is the placenta associated with the donor.
110. The computer system of item 110 wherein the stem cells from the second origin constitute a plurality of stem cell transplant units.
111. The computer system of item 110 wherein each stem cell transplant unit in said plurality of stem cell transplant units comprises between 25 x 10⁷ nucleated cells and 50 x 10⁷ nucleated cells.
112. The computer system of item 110 wherein each stem cell transplant unit in said plurality of stem cell transplant units comprises between 50 x 10⁷ nucleated cells and 75 x 10⁷ nucleated cells.
113. The computer system of item 110 wherein each stem cell transplant unit in said plurality of stem cell transplant units comprises between 75 x 10⁷ nucleated cells and 200 x 10⁷ nucleated cells.
114. The computer system of item 104 wherein said customer relationship management database is a flat file, a relational database, an on-line analytical processing database, or a hierarchical on-line analytical processing data cube.
115. The computer system of item 105 wherein said stem cell database comprises a flat file, a relational database, an on line analytical processing database, or a hierarchical on-line analytical processing data cube.
116. The computer system of item 104 wherein said customer relationship management database does not have an explicitly defined hierarchy.
117. The computer system of item 105 wherein said stem cell database does not have an explicitly defined hierarchy.
118. The computer system of claim 104 wherein said customer relationship management database includes a relational star schema.
119. The computer system of item 105 wherein said stem cell database includes a relational star schema.
120. The computer system of item 105 wherein said stem cell tracking module further includes:
   instructions for receiving type information of a patient; and
   instructions for searching through said plurality of stem cell units for a stem cell unit having type information that matches the type information of the patient.
121. The computer system of item 120 wherein said stem cell tracking module further includes instructions for receiving a search privilege, and wherein said instructions for searching are not performed when said search privilege is not received.
122. The computer system of item 120 wherein, when a match is found between a stem cell unit in said plurality of stem cell units and said patient, the stem cell tracking module further comprises granting the matching stem cell units to said patient when said matching stem cell units are public.
123. The computer system of item 120 wherein the type information is human leukocyte antigen type.
124. A computer system comprising:
   a customer relationship management database comprising information about a plurality of donors;
   a customer relationship management application that includes:
      (i) instructions for enrolling a donor in said customer relationship management database;
      (ii) instructions for uniquely associating a collection identifier number to said donor; and
   a stem cell database comprising information about a plurality of stem cell units; and
   a stem cell tracking module, the stem cell tracking module comprising a data entry routine that comprises:
      (i) instructions for receiving said collection identifier number;
      (ii) instructions for receiving a characterization of a stem cell unit from said donor, wherein said characterization includes a characterization of stem cells from at least two different origins of said donor; and
      (iii) instructions for storing said characterization of stem cells in said stem cell database.
125. A computer readable medium having computer-executable instructions for performing the steps of the method of item 1.
126. A computer program product for use in conjunction with a computer system, the computer program product comprising a computer readable storage medium and a computer program mechanism embedded therein, the computer program mechanism comprising a plurality of data records, wherein
   one or more respective data records in the plurality of data records comprises:
   a collection identifier number that uniquely corresponds to a stem cell donation;
   a cord blood cell count associated with the stem cell donation; and
   a placenta blood cell count associated with the stem cell donation.
127. The computer program product of item 126 wherein the stem cell donation originates from a mammal and the placental blood cell count represents a number of cells obtained from a post-partum placenta of the mammal after said placenta has been exsanguinated and perfused.
128. A computer program product for use in conjunction with a computer system, the computer program product comprising a computer readable storage medium and a computer program mechanism embedded therein, the computer program mechanism comprising a plurality of data records, wherein
   one or more respective data records in the plurality of data records comprises:
   a cord blood cell count associated with a stem cell donation;
   a placenta blood cell count associated with the stem cell donation; and
   an indication of at least two stem cell transplant units in said stem cell donation.
129. The computer program product of item 128 wherein the stem cell donation originates from a mammal and the placental blood cell count represents a number of cells obtained from a post-partum placenta of the mammal after said placenta has been exsanguinated and perfused.
130. A computer program product for use in conjunction with a computer system, the computer program product comprising a computer readable storage medium and a computer program mechanism embedded therein, the computer program mechanism comprising:
   a customer relationship management database comprising information about a plurality of donors;
   a customer relationship management application that includes:
      (i) instructions for enrolling a donor in said customer relationship management database;
      (ii) instructions for uniquely associating a collection identifier number to said donor; and
      (iii) instructions for transmitting said collection identifier number to a stem cell tracking module;
   a stem cell database comprising information about a plurality of stem cell units; and
   a stem cell tracking module, the stem cell tracking module comprising a data entry routine that comprises:
      (i) instructions for receiving a collection identifier number from said customer relationship management application;
      (ii) instructions for receiving a characterization of a stem cell unit from said donor, wherein said characterization includes a characterization of stem cells from at least two different origins of said donor; and
      (iii) instructions for storing said characterization of said stem cell unit from said donor in said stem cell database.
131. The computer program product of item 130 wherein said customer relationship management database and said stem cell database are on two different computers and wherein an access privilege is required to access said stem cell database.
132. The computer program product of item 130 wherein said stem cell database is a relational database, an on-line analytical processing database, or a hierarchical on-line analytical processing data cube and said information about said plurality of stem cell units can be searched for a stem cell unit having characteristics that match a query search.

## Claims

1. A method of maintaining a stem cell registry, said method comprising:
enrolling a donor in said stem cell registry;
receiving characterization information of one or more stem cell units from said donor,
wherein a stem cell unit in said one or more stem cell units is a population comprising stem cells, from a single donor, that have been derived from a single patient source, and comprises a plurality of stem cell transplant units, wherein each stem cell transplant unit in said plurality of stem cell transplant units is a portion of a stem cell unit, wherein a source of a first stem cell unit in said plurality of stem cell units differs from a source of a second stem cell unit in said plurality of stem cell units, and wherein
the first stem cell unit in said plurality of stem cell units from said donor comprises stem cells obtained from a post-partum placenta of the donor that has been exsanguinated and perfused, wherein said stem cells are OCT-4+ and ABC-p+; and
recording the characterization information about said plurality of stem cell transplant units in said stem cell registry;
wherein said stem cell registry comprises information about a plurality of stem cell units, the method further comprising:
obtaining type information of a patient; and
searching through said plurality of stem cell units for a stem cell unit having type information that matches the type information of the patient, wherein
when a match is found between a stem cell unit in said plurality of stem cell units and said patient, the method further comprises:
(i) allocating at least one stem cell transplant unit from the matching stem cell unit in said plurality of stem cell units to said patient; and
(ii) decrementing a number of stem cell transplant units of the matching stem cell unit available in the stem cell registry by the number of stem cell transplant units allocated to the patient.

2. The method of claim 1, wherein said enrolling comprises storing donor identification information about said donor in said stem cell registry.

3. The method of claim 1 wherein said step of receiving the characterization information comprises :
receiving a nucleated cell count for a stem cell transplant unit in said plurality of stem cell transplant units,
receiving a leukocyte count for a stem cell transplant unit in said plurality of stem cell transplant units,
receiving a mononuclear cell count for a stem cell transplant unit in said plurality of stem cell transplant units,
receiving a CD34-positive cell count for a stem cell transplant unit in said plurality of stem cell transplant units,
receiving a CD3-positive cell count for a stem cell transplant unit in said plurality of stem cell transplant units,
receiving a colony-forming cell assay for a stem cell transplant unit in said plurality of stem cell transplant units,
receiving an infectious disease assay for a stem cell transplant unit in said plurality of stem cell transplant units, or
receiving an HLA blood type of a stem cell transplant unit in said plurality of stem cell transplant units.

4. The method of claim 1, wherein said receiving said characterization information comprises separately characterization information of each stem cell transplant unit in said plurality of stem cell transplant units.

5. The method of claim 1 wherein
said enrolling said donor in said stem cell registry comprises receiving a fee from said donor, the method further comprising:
receiving a request from said donor or a family member of said donor for a stem cell transplant unit in said plurality of stem cell transplant units; and
allocating said stem cell transplant unit to said donor or said family member of said donor.

6. A computer program product for use in conjunction with a computer system, the computer program product comprising a computer readable storage medium and a computer program mechanism embedded therein, the computer program mechanism comprising:
a stem cell database comprising information about stem cells; and
a stem cell tracking module, the stem cell tracking module comprising a data entry routine that comprises:
instructions for enrolling a donor in said stem cell database;
instructions for receiving characterization information of one or more stem cell units that originate from said donor, wherein a stem cell unit in said one or more stem cell units is a population comprising stem cells, from a single donor, that have been derived from a single patient source, and comprises a plurality of stem cell transplant units, wherein each stem cell transplant unit in said plurality of stem cell transplant units is a portion of a stem cell unit, and wherein a source of a first stem cell unit in said plurality of stem cell units differs from a source of a second stem cell unit in said plurality of stem cell units, and wherein the first stem cell unit in said plurality of stem cell units from said donor comprises stem cells obtained from a post-partum placenta of the donor that has been exsanguinated and perfused, wherein said stem cells are OCT-4+ and ABC-p+; and
instructions for recording said characterization information in said stem cell database;
wherein the stem cell tracking module further includes a data query routine that comprises :
instructions for obtaining type information of a patient; and
instructions for searching through information about a plurality of stem cell units in said stem cell database for a stem cell unit having type information that matches the type information of the patient, wherein
when a match is found between a stem cell unit in said plurality of stem cell units and said patient, the data query routine further comprises:
instructions for allocating at least one stem cell transplant unit in the matching stem cell unit to said patient; and
instructions for decrementing a number of stem cell transplant units of the matching stem cell unit available in the stem cell registry by the number of stem cell transplant units allocated to said patient.

7. The computer program product of claim 6 wherein said instructions for receiving comprise:
instructions for receiving a nucleated cell count on a stem cell transplant unit in said plurality of stem cell transplant units,
instructions for receiving a leukocyte count for a stem cell transplant unit in said plurality of stem cell transplant units,
instructions for receiving a mononuclear cell count for a stem cell transplant unit in said plurality of stem cell transplant units,
instructions for receiving a CD34-positive cell count for a stem cell transplant unit in said plurality of stem cell transplant units,
instructions for receiving a CD3-positive cell count for a stem cell transplant unit in said plurality of stem cell transplant units,
instructions for receiving a colony-forming cell assay for a stem cell transplant unit in said plurality of stem cell transplant units,
instructions for receiving the results of an infectious disease assay for a stem cell transplant unit in said plurality of stem cell transplant units, or
instructions for receiving the results of an HLA blood type assay of a stem cell transplant unit in said plurality of stem cell transplant units.

8. The computer program product of claim 6, wherein
said instructions for enrolling said donor in said stem cell database comprise instructions for receiving a fee from said donor, and wherein the stem cell tracking module further includes a data query routine that comprises:
instructions for receiving a request from said donor or a family member for a stem cell transplant unit in said plurality of stem cell transplant units; and
instructions for allocating a stem cell transplant unit in said plurality of stem cell transplant units to said donor or said family member of said donor.

9. A computer system comprising:
a central processing unit;
a memory, coupled to the central processing unit, the memory storing:
a stem cell database comprising information about stem cell units; and
a stem cell tracking module, the stem cell tracking module comprising a data entry routine that comprises:
instructions for enrolling a donor in said stem cell database;
instructions for receiving characterization information of one or more stem cell units that originate from said donor, wherein a stem cell unit in said one or more stem cell units is a population comprising stem cells, from a single donor, that have been derived from a single patient source, and comprises a plurality of stem cell transplant units, wherein each stem cell transplant unit in said plurality of stem cell transplant units is a portion of a stem cell unit, and wherein a source of a first stem cell unit in said plurality of stem cell units differs from a source of a second stem cell unit in said plurality of stem cell units, and wherein the first stem cell unit in said plurality of stem cell units from said donor comprises stem cells obtained from a post-partum placenta of the donor that has been exsanguinated and perfused, wherein said stem cells are OCT-4+ and ABC-p+; and
instructions for recording said characterization information in said stem cell database;
wherein the stem cell tracking module further includes a data query routine that comprises:
instructions for obtaining type information of a patient; and
instructions for searching through information about a plurality of stem cell units that is stored in said stem cell database for a stem cell unit having type information that matches the type information of the patient, wherein
when a match is found between a stem cell unit in said plurality of stem cell units and said patient, the data query routine further comprises:
instructions for allocating at least one stem cell transplant unit from the matching stem cell unit in said plurality of stem cell units to said patient; and
instructions for decrementing a number of stem cell transplant units of the matching stem cell unit available in the stem cell registry by the number of stem cell transplant units allocated to the patient.

10. The method of claim 1, the computer program product of claim 6, or the computer system of claim 9 wherein each stem cell transplant unit in said plurality of stem cell transplant units comprises between 25 x 10⁷ nucleated cells and 50 x 10⁷ nucleated cells, or wherein each stem cell transplant unit in said plurality of stem cell transplant units comprises between 50 x 10⁷ nucleated cells and 75 x 10⁷ nucleated cells, or wherein each stem cell transplant unit in said plurality of stem cell transplant units comprises between 75 x 10⁷ nucleated cells and 200 x 10⁷ nucleated cells.

11. The computer program product of claim 6, or the computer system of claim 9, wherein said instructions for enrolling comprise instructions for storing donor identification information about said donor in said stem cell database.

12. The method of claim 1, the computer program product of claim 6, or the computer system of claim 9 wherein the at least one stem cell transplant units from the matching stem cell unit allocated to said patient is a function of the number of nucleated stem cells in each allocated stem cell transplant unit.

13. The method, the computer program product, or the computer system of claim 12 wherein the at least one stem cell transplant units from the matching stem cell unit allocated to said patient is also a function of the weight of the patient.

14. The method, the computer program product, or the computer system of claim 13 wherein between 0.5 x 10⁷ and 200 x 10⁷ nucleated cells per kilogram of patient body weight are allocated to said patient, or wherein between 1 x 10⁷ and 50 x 10⁷ nucleated cells per kilogram of patient body weight are allocated to said patient.

15. The method, the computer program product, or the computer system of claim 13 wherein the at least one stem cell transplant units from the matching stem cell unit allocated to said patient is also a function of the age of the patient.

16. The method of claim 1, the computer program product of claim 6, or the computer system of claim 9 wherein said donor is mammalian, preferably wherein the donor is human.

17. The method of claim 1, the computer program product of claim 6, or the computer system of claim 9 wherein said stem cell database comprises information about at least 100 stem cell units, or wherein said stem cell database comprises information about at least 1000 stem cell units.

18. The computer system of claim 9 wherein said instructions for receiving a characterization of said plurality of stem cell transplant units comprise:
instructions for receiving a nucleated cell count for a stem cell transplant unit in said plurality of stem cell transplant units,
instructions for receiving a leukocyte count for a stem cell transplant unit in said plurality of stem cell transplant units,
instructions for receiving a mononuclear cell count for a stem cell transplant unit in said plurality of stem cell transplant units,
instructions for receiving a CD34-positive cell count for a stem cell transplant unit in said plurality of stem cell transplant units,
instructions for receiving a CD3-positive cell count for a stem cell transplant unit in said plurality of stem cell transplant units,
instructions for receiving a colony-forming cell assay for a stem cell transplant unit in said plurality of stem cell transplant units,
instructions for receiving the results of an infectious disease assay for a stem cell transplant unit in said plurality of stem cell transplant units, or
instructions for receiving the results of an HLA blood type assay of a stem cell transplant unit in said plurality of stem cell transplant units.

19. The computer program product of claim 6, or computer system of claim 9, the data entry routine further comprising:
instructions for receiving a characterization of each stem cell transplant unit in said plurality of stem cell transplant units.

20. The computer system of claim 9, wherein said instructions for receiving a characterization comprises instructions for receiving a separate characterization of each stem cell transplant unit in said plurality of stem cell transplant units.

21. The computer system of claim 9, wherein
said instructions for enrolling said donor in said stem cell database comprise
instructions for receiving a fee from said donor, and wherein the stem cell tracking module further includes a data query routine that comprises:
instructions for receiving a request from said donor or a family member for one or more stem cell transplant units in said plurality of stem cell transplant units; and
instructions for allocating a stem cell transplant unit in said plurality of stem cell transplant units to said donor or said family member of said donor.

22. The computer system of claim 21 wherein at least one stem cell transplant unit in said plurality of stem cell transplant units remains after the allocation of the one or more stem cell transplant units.

23. The computer program product of claim 6, wherein the computer program mechanism further comprises a plurality of data records, wherein
one or more respective data records in the plurality of data records comprises:
a collection identifier number that uniquely corresponds to a stem cell donation;
a cord blood cell count associated with the stem cell donation; and
a placenta blood cell count associated with the stem cell donation.
